(19) **Europäisches Patentamt / European Patent Office / Office européen des brevets**

(11) **EP 1 225 234 B1**

(12) **EUROPEAN PATENT SPECIFICATION**

(45) Date of publication and mention
of the grant of the patent:
**07.11.2007 Bulletin 2007/45**

(51) Int Cl.:
***C12Q 1/68*** (2006.01)

(21) Application number: **02250379.1**

(22) Date of filing: **21.01.2002**

(54) **A method for sequencing nucleic acids**

Verfahren zur Sequenzierung von Nukleinsäuren

Procédé de séquencage d'acides nucléiques

(84) Designated Contracting States:
**DE FR GB**

(30) Priority: **20.01.2001 US 262973 P**
**16.01.2002 US 52926**

(43) Date of publication of application:
**24.07.2002 Bulletin 2002/30**

(73) Proprietor: **Agilent Technologies, Inc.**
**Santa Clara, CA 95051 (US)**

(72) Inventor: **Sampson, Jeffrey R.**
**Burlingame, CA 94010 (US)**

(74) Representative: **Powell, Stephen David et al**
**WILLIAMS POWELL**
**Morley House**
**26-30 Holborn Viaduct**
**London EC1A 2BP (GB)**

(56) References cited:
**WO-A-97/42346** **US-A- 6 015 714**

- KASIANOWICZ JOHN J ET AL: "Characterization of individual polynucleotide molecules using a membrane channel" PROCEEDINGS OF THE NATIONAL ACADEMY OF SCIENCES OF USA, NATIONAL ACADEMY OF SCIENCE. WASHINGTON, US, vol. 93, November 1996 (1996-11), pages 13770-13773, XP002139846 ISSN: 0027-8424
- DEAMER D W ET AL: "Nanopores and nucleic acids: prospects for ultrarapid sequencing" TRENDS IN BIOTECHNOLOGY, ELSEVIER, AMSTERDAM, NL, vol. 18, no. 4, April 2000 (2000-04), pages 147-151, XP004194002 ISSN: 0167-7799

**Description**

The present invention relates to synthesising nucleic acids and in particular to the synthesis and amplification of unstructured nucleic acids for rapid sequencing.

Determining the nucleotide sequence of DNA and RNA in a rapid manner is a major goal of researchers in biotechnology, especially for projects seeking to obtain the sequence of entire genomes of organisms. In addition, rapidly determining the sequence of a nucleic acid molecule is important for identifying genetic mutations and polymorphisms in individuals and populations of individuals.

Nanopore sequencing is one method of rapidly determining the sequence of nucleic acid molecules. Nanopore sequencing is based on the property of physically sensing the individual nucleotides (or physical changes in the environment of the nucleotides i.e. electric current, physical force) within an individual single-stranded piece of DNA as it traverses through a nanopore. In principle, the sequence of a polynucleotide can be determined from a single molecule. However, in practice, it is preferred that a sequence is determined from a statistical average of data obtained from the passage of hundreds of molecules having the same sequence through one or more pores.

The use of membrane channels to characterize polynucleotides as the molecules pass through the small ion channels has been studied. Kasianowicz et al. (Proc. Natl. Acad. Sci. USA. 93:13770-3, 1996) used an electric field to force single stranded RNA and DNA molecules through a 2.6 nanometer diameter ion channel in a lipid bilayer membrane. The diameter of the channel permitted only a single strand of a nucleic acid polymer to traverse the channel at any given time. As the nucleic acid polymer traversed the channel, the polymer partially blocked the channel, resulting in a transient decrease of ionic current. Since the length of the decrease in current is directly proportional to the length of the nucleic acid polymer, Kasianowicz et al. (supra) were able to determine experimentally lengths of nucleic acids by measuring changes in the ionic current.

Baldarelli et al. (US Pat. 6,015,714) and Church et al. (US Pat. 5,795,782) describe the use of small pores (nanopores) to characterize polymers including DNA and RNA molecules on monomer by monomer basis. In particular, Baldarelli et al. (supra) characterize and sequence nucleic acid polymers by passing a nucleic acid through a channel (or pore). The channel is imbedded in an interface which separates two media. As the nucleic acid molecule passes through the channel, the nucleic acid alters an ionic current by blocking the channel. As the individual nucleotides pass through the channel, each base/nucleotide alters the ionic current in a manner which allows one to identify the nucleotide transiently blocking the channel, thereby allowing one to determine the nucleotide sequence of the nucleic acid molecule.

However, several technical problems limit the rate and accuracy of nanopore sequencing of nucleic acid polymers. One limitation is the rate at which the sequencing of a molecule is initiated. Since one end of a single nucleic acid molecule must enter the nanopore to initiate the sequencing, the rate is limited by the rate at which a nucleic acid molecule stochastically enters a nanopore. This rate limitation is imposed by the initiation of processing, and can be minimized by increasing the concentration of the polymer using amplification methods such as the polymerase chain reaction (PCR).

Another limitation to the rate of nanopore sequencing of nucleic acids is due to the formation of intramolecular base pairing between regions of complementarity (secondary structure) within a single strand of nucleic acid being sequenced. The formation of secondary structure limits the ability of a nucleic acid molecule to pass through a nanopore, stalling the molecule in the nanopore, and therefore reduces the rate of sequencing.

Therefore, there is a need for improved methods of rapidly and accurately sequencing nucleic acid molecules.

In one aspect, the present invention provides an improved method of determining the sequence of a nucleic acid polymer using nanopore sequencing. The present invention generates nucleic acid polymers for nanopore sequencing having multiple tandem repeats of a sequence. A molecule having such tandem repeats reduces the influence of process initiation on the rate of nanopore sequencing. Without limitation to the theory, it is proposed that after an end of a nucleic acid molecule containing such tandem repeats has entered a nanopore, process initiation is not a factor in the rate of sequencing of the other repeated sequences. Therefore, the overall sequencing throughput will be proportional to the number of tandem repeats in one molecule. In addition, over-sampling of a sequence tandemly repeated within one molecule reduces the variability in sequencing data caused by variations in the pores if multiple pores are used.

In a preferred embodiment, nucleic acid molecules having tandemly repeated sequences are synthesized enzymatically using a circular template. Preferably the template is single-stranded, although double stranded circular nucleic acid molecules may also be used.

In an embodiment, the present invention provides an improved method of sequencing that increases the rate of nanopore sequencing by also reducing secondary structure in nucleic acid molecules to be sequenced. Nucleic acid molecules with reduced secondary structure ("unstructured nucleic acids"; UNA) are generated by enzymatically incorporating modified nucleotide triphosphates that have a reduced ability to form base pairs with complementary modified and unmodified nucleotides. Preferably, the UNAs are generated from a template containing complementary unmodified nucleotides. However, it is within the scope of the present invention for the template to contain other modified nucleotide complements that do form base pairs with the UNA in order for the template to be used by enzymes for nucleotide incorporation into UNAs.

In a preferred embodiment, unstructured nucleic acids are synthesized enzymatically by incorporating nucleotide precursors which cannot form base pairs with one form of a complementary nucleotide incorporated into the unstructured nucleic acid and does form base pairs with another form of a complementary nucleotide, preferably present in a template molecule. In a particularly preferred embodiment, unstructured nucleic acids are enzymatically synthesized by incorporating triphosphate forms of 2-aminoadenosine, 2-thiothymidine, inosine, pyrrolo-pyrimidine and combinations therein.

Preferably, the present invention provides an improved method of nanopore sequencing by generating a nucleic acid molecule to be sequenced that has tandem repeats of a sequence, and also has modified nucleotides with a reduced ability to form base pairs with modified and/or unmodified complements. Modified nucleotides and complements having a reduced ability to form base pairs with each other reduces or eliminates the secondary structure (intramolecular base pairing) that may form between regions of complementarity within a nucleic acid molecule. Therefore, a molecule with reduced (or no) secondary structure will pass through a nanopore more readily than a molecule with secondary structure.

In a preferred embodiment, unstructured nucleic acids to be sequenced by nanopore sequencing are enzymatically synthesized using a circular template by incorporating nucleotide precursors which have a reduced ability to form base pairs with one form of a complementary nucleotide also incorporated into the unstructured nucleic acid but are still capable of forming base pairs with another form of a complementary nucleotide, preferably present in the circular template. In a particularly preferred embodiment, unstructured nucleic acids are enzymatically synthesized from a circular template by incorporating triphosphate forms of 2-aminoadenosine, 2-thiothymidine, inosine, pyrrolo-pyrimidine and combinations therein.

In yet another aspect, the present invention provides a method for synthesizing a nucleic acid molecule with reduced levels of secondary structure and preferably with multiple tandem repeats of a sequence.

## Definitions

"Sequencing": The term "sequencing" as used herein means determining the sequential order of nucleotides in a nucleic acid molecule. Sequencing as used herein includes in the scope of its definition, determining the nucleotide sequence of a nucleic acid in a *de novo* manner in which the sequence was previously unknown. Sequencing as used herein also includes in the scope of its definition, determining the nucleotide sequence of a nucleic acid where in the sequence was previously known. Sequencing nucleic acid molecule whose sequence was previously known may be used to identify a nucleic acid molecule, to confirm a nucleic acid sequence, or to search for polymorphisms and genetic mutations.

"Secondary Structure": Secondary structure as used herein means the intramolecular base pairing of regions of self-complementarity in a nucleic acid molecule. Secondary structure forms in DNA and RNA molecules. Non-limiting examples of secondary structures include hairpins, loops, bulges, duplexes, junctions, stems, pseudoknots, triple helices, H-DNA, hammerheads, and self-splicing ribozymes. For purposes of the present invention, secondary structure includes higher order structures such as tertiary structures.

"Modified Nucleotide": Nucleic acid bases may be defined for purposes of the present invention as nitrogenous bases derived from purine or pyrimidine. Modified bases (excluding A, T, G, C, and U) include for example, bases having a structure derived from purine or pyrimidine (i.e. base analogs). For example without limitation, a modified adenine may have a structure comprising a purine with a nitrogen atom covalently bonded to C6 of the purine ring as numbered by conventional nomenclature known in the art. In addition, it is recognized that modifications to the purine ring and/or the C6 nitrogen may also be included in a modified adenine. A modified thymine may have a structure comprising at least a pyrimidine, an oxygen atom covalently bonded to the C4 carbon, and a C5 methyl group. Again, it is recognized by those skilled in the art that modifications to the pyrimidine ring, the C4 oxygen and/or the C5 methyl group may also be included in a modified adenine. Derivatives of uracil may have a structure comprising at least a pyrimidine, an oxygen atom covalently bonded to the C4 carbon and no C5 methyl group. For example without limitation, a modified guanine may have a structure comprising at least a purine, and an oxygen atom covalently bonded to the C6 carbon. A modified cytosine has a structure comprising a pyrimidine and a nitrogen atom covalently bonded to the C4 carbon. Modifications to the purine ring and/or the C6 oxygen atom may also be included in modified guanine bases. Modifications to the pyrimidine ring and/or the C4 nitrogen atom may also be included in modified cytosine bases.

Analogs may also be derivatives of purines without restrictions to atoms covalently bonded to the C6 carbon. These analogs would be defined as purine derivatives. Analogs may also be derivatives of pyrimidines without restrictions to atoms covalently bonded to the C4 carbon. These analogs would be defined as pyrimidine derivatives. The present invention includes purine analogs having the capability of forming stable base pairs with pyrimidine analogs without limitation to analogs of A, T, G, C, and U as defined. The present invention also includes purine analogs not having the capability of forming stable base pairs with pyrimidine analogs without limitation to analogs of A, T, G, C, and U.

In addition to purines and pyrimidines, modified bases or analogs, as those terms are used herein, include any compound that can form a hydrogen bond with one or more naturally occurring bases or with another base analog. Any compound that forms at least two hydrogen bonds with T (or U) or with a derivative of T or U is considered to be an analog of A or a modified A. Similarly, any compound that forms at least two hydrogen bonds with A or with a derivative of A is considered to be an analog of T (or U) or a modified T or U. Similarly, any compound that forms at least two hydrogen bonds with G or with a derivative of G is considered to be an analog of C or a modified C. Similarly, any compound that forms at least two hydrogen bonds with C or with a derivative of C is considered to be an analog of G or a modified G. It is recognized that under this scheme, some compounds will be considered for example to be both A analogs and G analogs.

"Hybridization": Hybridization as used herein means the formation of hydrogen-bonded base pairs between two regions having substantially complementary sequences to form a duplex. Duplex formation may be intermolecular or intramolecular. Two complementary sequences do not have to be 100% complementary for duplex formation. Certain mismatches may be tolerated for hybridization to occur. Conditions that promote duplex formation or hinder duplex formation are well-known to those of ordinary skill in the art. It is recognized that hybridization includes in its definition, transiently stable duplex which are stable long enough to be detected and/or to allow a biological process to occur (e.g. primer extension).

A stable base pair is defined as two bases that can interact through the formation of at least two hydrogen bonds. Alternatively or additionally, a stable base pair may be defined as two bases that interact through at least one, preferably two, hydrogen bonds that promote base stacking interactions and therefore, promotes duplex stability.

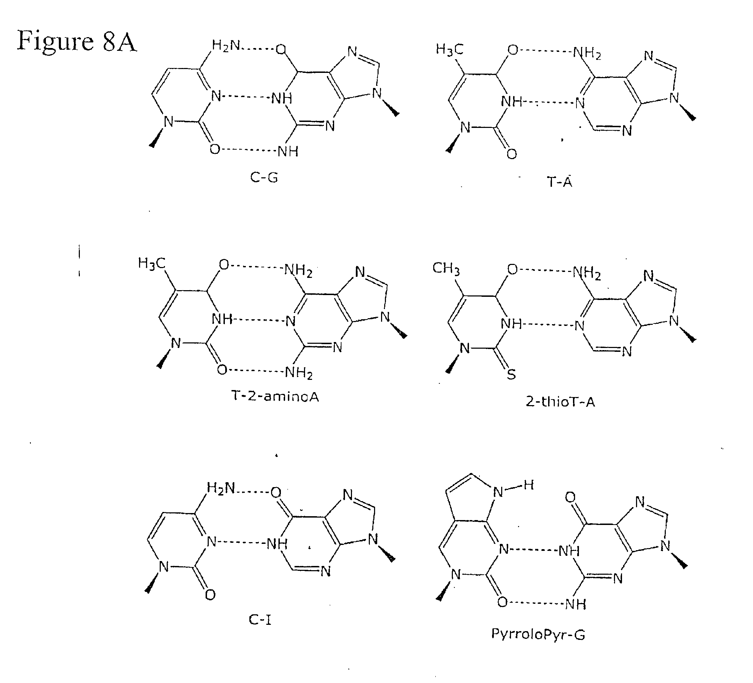

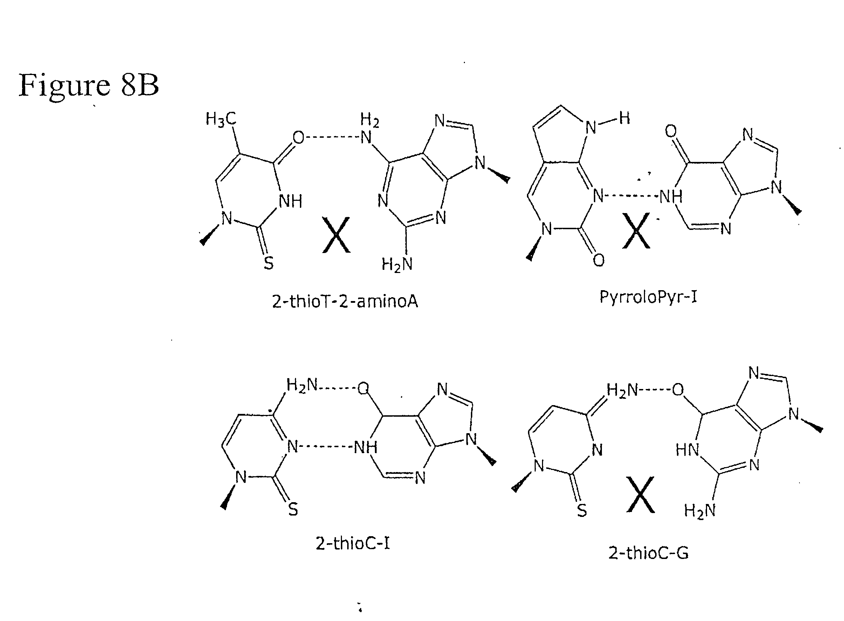

"Complementary": Complementary bases are defined according to the Watson-Crick definition for base pairing. Adenine base is complementary to thymine base and forms a stable base pair. Guanine base is complementary to cytosine base and forms a stable base pair. The base pairing scheme is depicted in Figure 8. Complementation of modified base analogs is defined according to the parent nucleotide. Complementation of modified bases does not require the ability to form stable hydrogen bonded base pairs. In other words, two modified bases may be complementary but may not form a stable base pair. Complementation of base analogs which are not considered derivatives of A, T, G, C or U is defined according to an ability to form a stable base pair with a base or base analog. For example, a particular derivative of C (i.e. 2-thiocytosine) may not form a stable base pair with G, but is still considered complementary.

"Naturally occurring bases": Naturally occurring bases are defined for the purposes of the present invention as adenine (A), thymine (T), guanine (G), cytosine (C), and uracil (U). The structures of A, T, G and C are shown in Figure 8. For RNA, uracil (U) replaces thymine. Uracil (structure not shown) lacks the 5-methyl group of T. It is recognized that certain modifications of these bases occur in nature. However, for the purposes of the present invention, modifications of A, T, G, C, and U that occur in nature are considered to be non-naturally occurring. For example, 2-aminoadenosine is found in nature, but is not a "naturally occurring" base as that term is used herein. Other non-limiting examples of modified bases that occur in nature but are considered to be non-naturally occurring are 5-methylcytosine, 3-methyladenine, O(6)-methylguanine, and 8-oxoguanine.

Figure 1. Figure 1 depicts the sequencing of nucleic acid molecules using a single pore and using multiple pores.

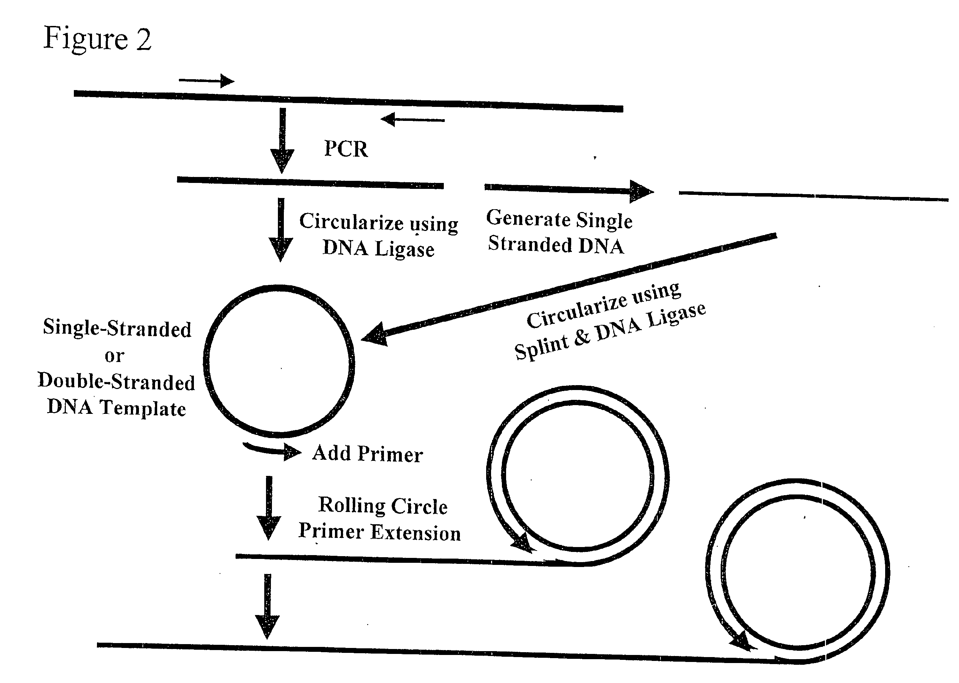

Figure 2. Figure 2 depicts the enzymatic synthesis of tandemly repeated single-stranded DNA molecules from a either a single-stranded or double-stranded circular template for nanopore sequencing.

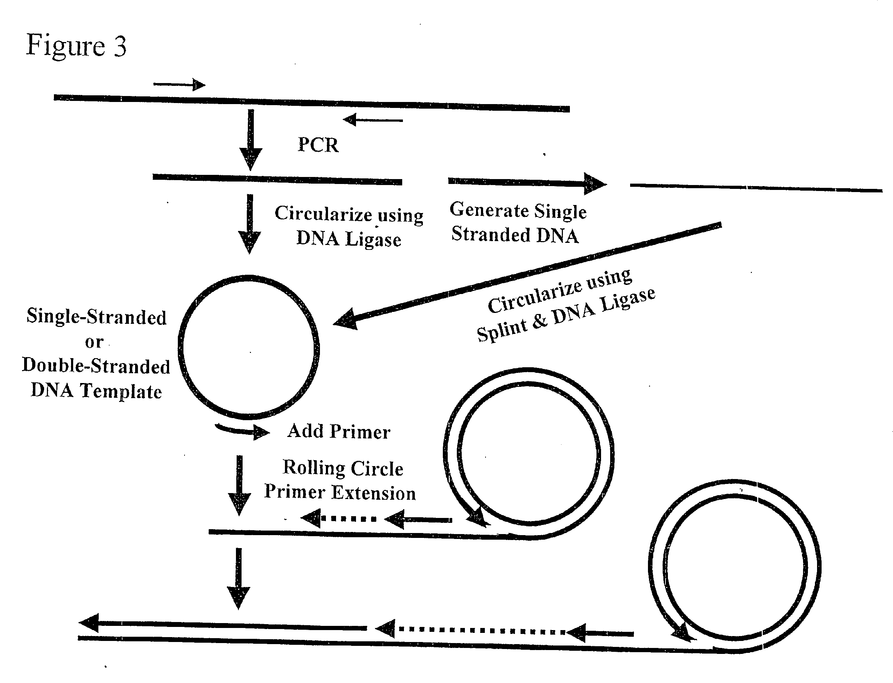

Figure 3. Figure 3 depicts the enzymatic synthesis of tandemly repeated double-stranded DNA molecules from either a single-stranded or double-stranded circular template for nanopore sequencing.

Figure 4. Figure 4 depicts the enzymatic synthesis of tandemly repeated single stranded RNA molecules from a single-stranded circular template.

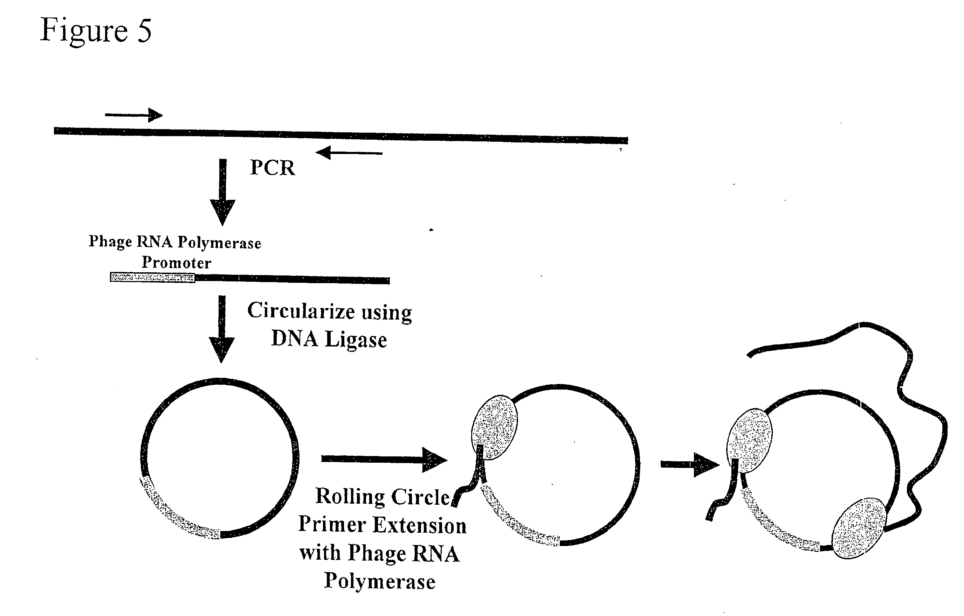

Figure 5. Figure 5 depicts the enzymatic synthesis of tandemly repeated single stranded RNA molecules from a double-stranded circular template.

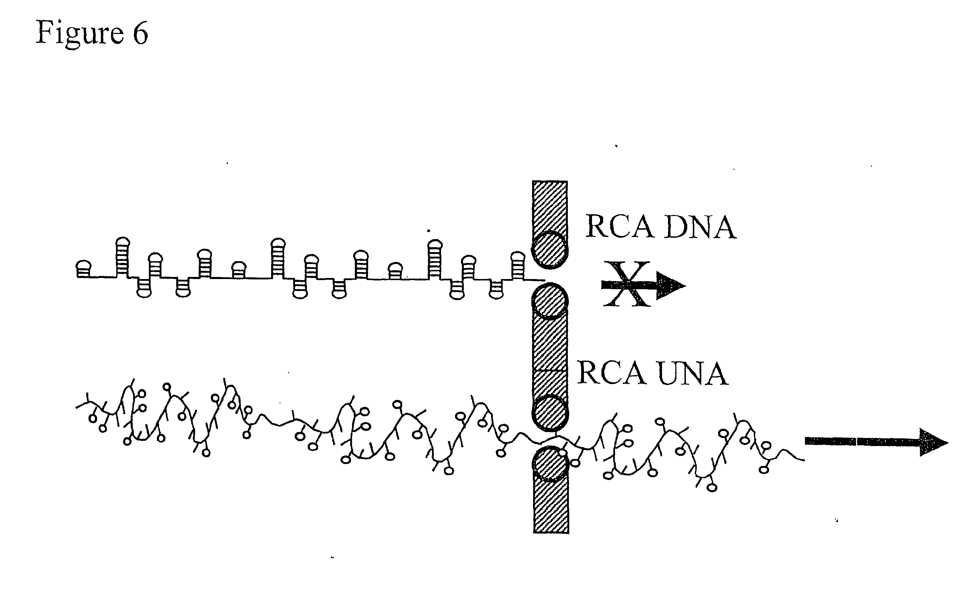

Figure 6. Figure 6 depicts nanopore sequencing of nucleic acid molecules with secondary structure and nanopore sequencing of unstructured nucleic acid molecules.

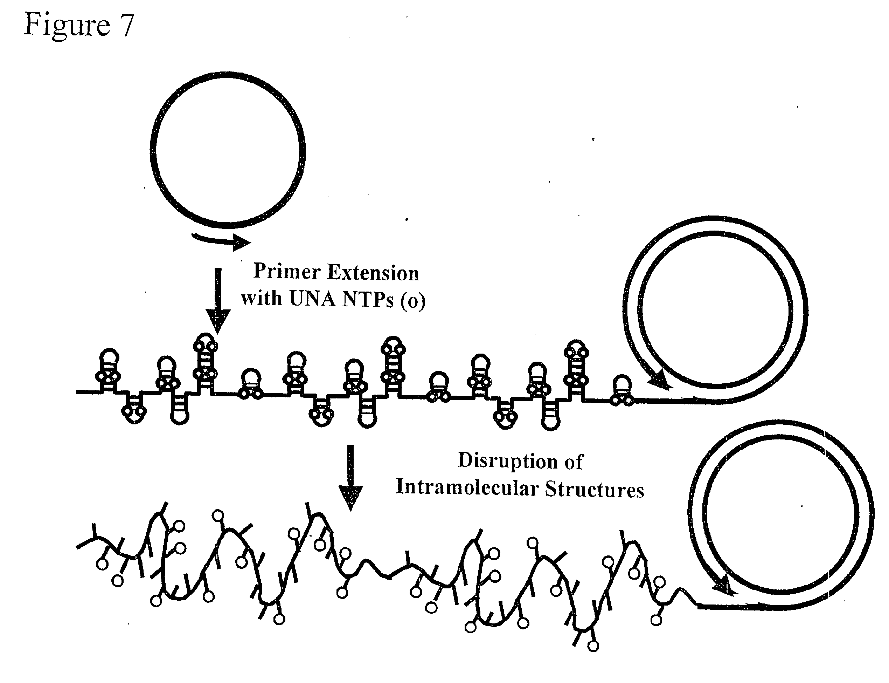

Figure 7. Figure 7 depicts the structure of complementary bases forming base pairs and the disruption of the complementary bases pairs by the UNA nucleotides.

Figure 8. Figure 8, panels A and B, depict the structure of complementary bases forming base pairs and complementary bases which do not form base pairs.

The present invention provides improved systems and methods for amplifying and sequencing nucleic acid polymers. Generally, the present invention utilizes nanopore sequencing, nucleic acid amplification and modified nucleotides to amplify and sequence nucleic acid polymers at rates and with accuracies that are greater than current conventional nucleic acid sequencing techniques.

Nanopore sequencing of nucleic acids has been described (USP 5,795,782 to Church et al.; USP 6,015,714 to Baldarelli et al.,).

These methods of nanopore sequencing of polymers, including nucleic acids, have several disadvantages which limit the rate of sequencing and reduce the accuracy of the sequencing information. One limitation is the rate at which the sequencing of a molecule is initiated. Since one end of a single nucleic acid molecule must enter the nanopore to initiate the sequencing, the rate is limited by the rate at which a nucleic acid molecule stochastically enters a nanopore. This rate limitation is imposed by the initiation of processing, and can be minimized by increasing the concentration of the polymer using amplification methods such as the polymerase chain reaction (PCR).

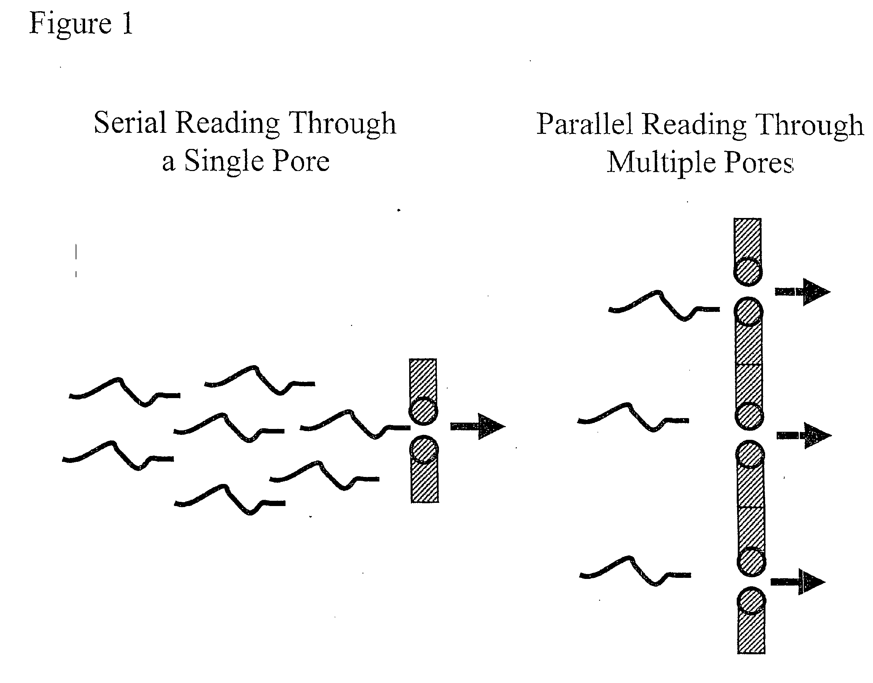

Additionally or alternatively, after amplification of the nucleic acid molecules, the nucleic acids can be sequenced in parallel using multiple pores (Figure 1). If multiple pores are used, each pore must be produced with precise reproducibility and consistency to ensure that data obtained from all the pores are consistent. For example, variable pore sizes may create undesirable noise in the sequencing data. Furthermore, the accuracy of nanopore sequencing is dependent on the signal-to-noise ratio obtained during sequencing. Thus, the signal-to-noise ratio can be improved by increasing the number of nucleic acid molecules sequenced through one or more nanopores.

Another limitation to the rate of nanopore sequencing of nucleic acids is due to the formation of intramolecular base pairing between regions of complementarity (secondary structure) within a single strand of nucleic acid being sequenced. The formation of secondary structure limits the ability of a nucleic acid molecule to pass through a nanopore, stalling the molecule in the nanopore, and therefore reduces the rate of sequencing.

In one aspect, the present invention provides an improved method of determining the sequence of a nucleic acid polymer using nanopore sequencing. The present invention generates nucleic acid polymers for nanopore sequencing having multiple tandem repeats of a sequence. A molecule having such tandem repeats reduces the influence of process initiation on the rate of nanopore sequencing. Without limitation to the theory, it is proposed that after an end of a nucleic acid molecule containing such tandem repeats has entered a nanopore, process initiation is not a factor in the rate of sequencing of the other repeated sequences. Therefore, the overall sequencing throughput will be proportional to the number of tandem repeats in one molecule. In addition, over-sampling of a sequence tandemly repeated within one molecule reduces the variability in sequencing data caused by variations in the pores if multiple pores are used.

In another aspect, the present invention provides an improved method of sequencing that increases the rate of nanopore sequencing by reducing secondary structure in nucleic acid molecules to be sequenced. Nucleic acid molecules with reduced secondary structure ("unstructured nucleic acids"; UNA) are generated by enzymatically incorporating modified nucleotide triphosphates that have a reduced ability to form base pairs with complementary modified and unmodified nucleotides. Preferably, the UNAs are generated from a template containing complementary unmodified nucleotides. However, it is within the scope of the present invention for the template to contain other modified nucleotide complements that do form base pairs with the UNA in order for the template to be used by enzymes for nucleotide incorporation into UNAs.

In yet another aspect, the present invention provides an improved method of nanopore sequencing by generating a nucleic acid molecule to be sequenced that has tandem repeats of a sequence, and also has modified nucleotides with a reduced ability to form base pairs with modified and/or unmodified complements. Modified nucleotides and complements having a reduced ability to form base pairs with each other reduces or eliminates the secondary structure (intramolecular base pairing) that may form between regions of complementarity within a nucleic acid molecule. Therefore, a molecule with reduced (or no) secondary structure will pass through a nanopore more readily than a molecule with secondary structure.

The present invention also describes a method for synthesizing a nucleic acid molecule with reduced levels of secondary structure and preferably with multiple tandem repeats of a sequence.

In a preferred embodiment, nucleic acid molecules for nanopore sequencing with tandem repeats are generated enzymatically from a circular template containing one or more copies of the complememary sequence. Preferably, the template is a single stranded. However, double stranded circular nucleic acids (e.g. DNA) may be denatured and optionally cleaved on one strand to create a single stranded template. The template is used in a primer-dependent DNA or RNA polymerase reaction which synthesizes a nucleic acid having complementary sequences. In the presence of nucleotide precursors, the polymerization reaction will continue around the circular template, and will then displace the primer and subsequent double stranded regions to continue the polymerization reaction. As the polymerase synthesizes a complement of the circular template, additional tandem repeats are added to the nascent polymer. Theoretically, there is no limit to the number of repeats which can be synthesized in a polymerization reaction using a circular template. However, in practice, the length of a polymerization reaction product using a circular template is determined in part by the processivity of the enzyme used.

ROLLING CIRCLE AMPLIFICATION

In a particularly preferred embodiment of the present invention, nucleic acid molecules having multiple repeats of a sequence are generated by rolling circle amplification (RCA) for nanopore sequencing. RCA is an isothermal reaction that amplifies a nucleic acid molecule through primer extension using enzymatic methods, nucleotide precursors and a circularized template. Briefly, the method of RCA of tandem DNA molecules involves 1) providing a circular single-stranded nucleic acid template; 2) providing a primer having a sequence substantially complementary to a sequence present in the template, 3) annealing the primer to the template under suitable conditions; 4) contacting the primer: template hybrid with at least one nucleotide precursor and at least one enzyme characterized by the ability to polymerize the precursor into a polynucleotide in a primer-dependent manner under the conditions and for a time suitable for the formation of a polynucleotide such that the resulting polynucleotide has multiple repeats of a sequence substantially complementary to a sequence in the template.

The method of RCA of tandem RNA molecules involves 1) providing a circular single-stranded nucleic acid template having a sequence corresponding to a suitable RNA polymerase promoter; 2) providing an additional oligonucleotide having a sequence that is complementary to an RNA polymerase promoter region of the template; 3) annealing the promoter oligonucleotide to the template under suitable conditions; 4) contacting the promoter:template hybrid with at least one ribonucleotide precursor and at least one enzyme characterized by the ability to polymerize the ribonucleotide precursor into a poly-ribonucleotide in a promoter-dependent manner under the conditions and for a time suitable for the formation of a polyribonucleotide such that the resulting polyribonucleotide has multiple repeats of a sequence substantially complementary to the template sequence.

Alternatively, RCA of tandem RNA molecules can be performed by 1) providing a circular double-stranded nucleic acid template having a sequence corresponding to a suitable RNA polymerase promoter and 2) contacting the promoter:template hybrid with at least one ribonucleotide precursor and at least one enzyme characterized by the ability to polymerize the ribonucleotide precursor into a poly-ribonucleotide in a promoter-dependent manner under the conditions and for a time suitable for the formation of a polyribonucleotide such that the resulting polyribonucleotide has multiple repeats of a sequence substantially complementary to the template sequence.

RCA produces long (>10,000 nucleotides) single-stranded polynucleotides (RNA or DNA) corresponding to potentially over 100 tandem copies of a sequence complementary to the circular template. As a result, RCA targets would allow a single pore entry event to facilitate the reading of >100 copies of the target sequence.

Kool (USP 5, 714, 320) teaches a method of enzymatically synthesizing a nucleic acid molecule using a circular template which generates single stranded multimers complementary to a circular template. In a standard reaction, RCA requires a small amount of the circular template, primer, and polymerase enzyme, (i.e., only an effective catalytic amount for each component). Surprisingly, no auxiliary proteins need to be added to assist the polymerase. However, the present invention does not exclude the use of auxiliary proteins for use with a polymerizing enzyme. A relatively larger amount, (i.e., a stoichiometric amount) of the nucleotide triphosphates (or nucleotide precursors) is required. After the reaction, the mixture consists of a large amount of the product oligomer and only small amounts of the template, primer, and polymerase enzyme. Thus, the product is produced in relatively good purity, and can require only gel filtration or dialysis before use, depending on the application. Advantageously, the polymerase enzyme, the circular template, unreacted primer, and unreacted nucleotide triphosphates can be recovered for further use.

A. Circular Templates

Any method of producing circular single-stranded nucleic acid template molecules may be used in accordance with the present invention. Preferably circular templates are about 15-1500 nucleotides. More preferably, the circular templates are about 24-500 nucleotides, and most preferably, the circular templates are about 30-150 nucleotides. The nucleic acid template may be RNA or DNA, but preferably DNA. The nucleic acid template may containing any natural or non-natural base, sugar and/or backbone which permits a nucleotide polymerizing enzyme to synthesize a polynucleotide having a nucleotide sequence that is complementary to the sequence of the template. Preferably, the nucleic acid template comprises naturally-occurring deoxyribonucleic acids.

Construction of circular template.

To perform RCA, an isolated circular oligonucleotide template is provided. For a desired oligomer, a circular oligonucleotide template which is complementary in sequence to the desired oligonucleotide product can be prepared from a linear precursor, i.e., a linear precircle. The template linear precircle has a 3'- or 5'-phosphate group. If the desired oligonucleotide product sequence is short (i.e., less than about 20-30 bases), a double or higher multiple copy of the complementary sequence can be contained in the template circle. This is generally because enzymes cannot process circular sequences of too small a size. Typically, a circular template has about 15-1500 nucleotides, preferably about 24-500, and more preferably about 30-150 nucleotides. It is to be understood that the desired nucleotide product sequence can either be a sense, antisense, or any other nucleotide sequence.

Linear precircle oligonucleotides, from which the circular template oligonucleotides are prepared, can be made by any of a variety of procedures known for making DNA and RNA oligonucleotides. For example, the linear precircle can be synthesized by any of a variety of known techniques, such as enzymatic or chemical, including automated synthetic methods. Furthermore, the linear oligomers used as the template linear precircle can be synthesized by the rolling circle method of the present invention. Many linear oligonucleotides are available commercially, and can be phosphorylated on either end by any of a variety of techniques.

Linear precircle oligonucleotides can also be restriction endonuclease fragments derived from naturally occurring DNA sequence. Briefly, DNA isolated from an organism can be digested with one or more restriction enzymes. The desired oligonucleotide sequence can be isolated and identified by standard methods as described in Sambrook et al., A Laboratory Guide to Molecular Cloning, Cold Spring Harbor, N.Y. (1989). The desired oligonucleotide sequence can contain a cleavable site, or a cleavable site can be added to the sequence by ligation to a synthetic linker sequence by standard methods.

Linear precircle oligonucleotides can be purified by polyacrylamide gel electrophoresis, or by any number of chromatographic methods, including gel filtration chromatography and high performance liquid chromatography.

The present invention also provides several methods wherein the linear precircles are then ligated chemically or enzymatically into circular form. This can be done using any standard techniques that result in the joining of two ends of the precircle. Such methods include, for example, chemical methods employing known coupling agents such as BrCN plus imidazole and a divalent metal, N-cyanoimidazole with $ZnCl_2$, 1-(3-dimethylaminopropyl)-3 ethylcarbodiimide HCl, and other carbodiimides and carbonyl diimidazoles. Furthermore, the ends of a precircle can be joined by condensing a 5'-phosphate and a 3'-hydroxyl, or a 5'-hydroxyl and a 3'-phosphate. Enzymatic circle closure is also possible using DNA ligase or RNA ligase under conditions appropriate for these enzymes.

One enzymatic approach utilizes T4 RNA ligase, which can couple single-stranded DNA or RNA. This method is described in Tessier et al., Anal Biochem., 158, 171-178 (1986). Under high dilution, the enzyme ligates the two ends of an oligomer to form the desired circle. Alternatively, a DNA ligase can be used in conjunction with an adaptor oligomer under high dilution conditions.

Preferably, the method of forming the circular oligonucleotide template involves adapter directed coupling. Methods such as this are described in G. Prakash et al., J. Am. Chem. Soc., 114, 3523-3527 (1992), E. T. Kool, PCT Publication WO 92/17484, and E. Kanaya et al., Biochemistry, 25, 7423-7430 (1986).

This method includes the steps of: hybridizing a linear precursor having two ends to an adapter, i.e., a positioning oligonucleotide, to form an open oligonucleotide circle; joining the two ends of the open oligonucleotides circle to form the circular oligonucleotide template; and recovering the single-stranded circular oligonucleotide template. The positioning oligonucleotide is complementary to the two opposite ends of the linear precursor. The precursor and the adapter are mixed and annealed, thereby forming a complex in which the 5' and 3' ends of the precircle are adjacent. The adapter juxtaposes the two ends. This occurs preferentially under high dilution, i.e., no greater than about 100 micromolar, by using very low concentrations of adapter and precursor oligomers, or by slow addition of the adapter to the reaction mixture. These ends then undergo a condensation reaction, wherein the 5'-phosphate is coupled to the 3'-hydroxyl group or the 3'-phosphate is coupled to the 5'-hydroxyl group, after about 6-48 hours of incubation at about 4°-37° C. This occurs in a buffered aqueous solution containing divalent metal ions and BrCN at a pH of about 7.0. Preferably, the buffer is imidazole-HCl and the divalent metal is Ni, Zn, Mn, Co, Cu, Pb, Ca, or Mg. More preferably, the metals are Ni and Zn. Other coupling reagents that work include 1-(3-dimethylaminopropyl)-3 ethylcarbodiimide HCl, and other water-soluble carbodiimides, or any water-active peptide coupling reagent or esterification reagent.

The circular oligonucleotide template can be purified by standard techniques although this may be unnecessary. For example, if desired the circular oligonucleotide template can be separated from the positioning oligonucleotide by denaturing gel electrophoresis or melting followed by gel electrophoresis, size selective chromatography, or other appropriate chromatographic or electrophoretic methods. The isolated circular oligonucleotide can be further purified by standard techniques as needed.

Primer

The primer used in the rolling circle method is generally short, preferably containing about 4-50 nucleotides, and more preferably about 6-12 nucleotides. This primer is substantially complementary to part of the circular template, preferably to the beginning of the desired oligomer sequence. A substantially complementary primer has no more than about 1-3 mismatches while still maintaining sufficient binding to the template. The 3' end of the primer must be at least about 80%, preferably 100%, complementary to the circular template. There is no requirement that the 5' end be complementary, as it would not have to bind to the template. Although a portion of the primer does not have to bind to the circular template, about 4-12 nucleotides should be bound to provide for initiation of nucleic acid synthesis. The primer can be synthesized by any of the methods discussed above for the linear precircle oligomer, such as by standard solid-phase techniques. See, for example, S. L. Beaucage et al., Tetrahedron Lett., 22, 1859 (1981) (for DNA), and S. A. Scaringe et al., Nucleic Acids Res., 18, 5433 (1990) (for RNA).

When the sequence of the circular template is unknown, a mixture of primers may be used containing all possible nucleotide sequences of a given length. For example, random hexamer primers are commercially available and contain a mixture of all possible nucleic acid sequences having six nucleotides based on A, G, T and C ($4^6$=4096).

Primers containing modified nucleotides which are capable of hybridizing to a circular template may also be used in accordance with the present invention.

An effective amount of the primer is added to the buffered solution of an effective amount of the circular template under conditions to anneal the primer to the template. An effective amount of the primer is present at about 0.1-100 moles primer per mole of circular template, preferably 0.1-10. An effective amount of the circular template is that amount that provides for sufficient yield of the desired oligomer product. The effective amount of the circular template depends on the scale of the reaction, the size and sequence of circular template, and the efficiency of the specific rolling circle synthesis. Typically, the amount of the circular template is present at about a 1:5 to 1:20,000 ratio with the amount of desired oligomer product, i.e., 1-5000 fold amplification, preferably 1:50 to 1:5000 ratio.

Conditions

Conditions that promote annealing are known to those of skill in the art for both DNA-DNA compositions and DNA-RNA compositions and are described in Sambrook et al., cited supra. Once formed, the primed circular template is used to initiate synthesis of the desired oligomer or multimer.

Rolling circle synthesis

Rolling circle synthesis is initiated when nucleotide triphosphates and polymerase are combined with a primed circular template. At least two types of nucleotide triphosphate, along with an effective catalytic amount of the desired polymerase enzyme are added to the mixture of the primer and circular template. Amplified run-on synthesis then occurs: the polymerase starts at the primer, elongates it, and continues around the circle, making the desired oligonucleotide product sequence. It continues past the starting point, displacing the synthesized DNA (or RNA) as it goes, and proceeds many times around the circle. This produces a long single multimer strand which is made up of many end-to-end copies of the desired oligonucleotide product. The size of the multimer product can be about 60 to $5 \times 10^6$ nucleotides in length. More preferably, the multimer product is about 500-100,000 nucleotides in length.

The length of the multimer can be controlled by time, temperature, relative and absolute concentrations of enzyme, triphosphates, template, and primer. For example, longer periods of time, or lower concentrations of template, will tend to increase the average multimer length. The rolling circle method preferably uses only catalytic amounts of template, primer, and polymerase enzymes and stoichiometric amounts of the nucleotide triphosphates. Typically, the maximum size of multimer product is unlimited, however, often it is about $10^4$ -$10^6$ nucleotides in length.

More preferably, the template concentration is about 0.1 microM to about 1 mM, the primer concentration is about 0.1 microM to about 1 mM, and the triphosphate concentration is about 1 microM to about 1000 mM. The preferred molar ratio of triphosphate(s) to template is about 50:1 to about $10^7$:1. The preferred molar ratio of primer to template is about 0.1:1 to about 100:1. These preferred amounts, i.e., concentrations and molar ratios, refer to amounts of the individual components initially provided to the reaction mixture.

The preferred reaction time for the rolling circle synthesis is about 1 hour to about 3 days. Preferably, the temperature of the reaction mixture during the rolling circle synthesis is about 20°-90° C. For polymerase enzymes that are not thermally stable, such as DNA polymerase I and its Klenow fragment, and other nonengineered enzymes, the temperature of synthesis is more preferably about 20°-50° C. For thermostable polymerases, such as that from *Thermus aquaticus,* the temperature of synthesis is more preferably about 50°-100° C.

Oligomers may be radiolabeled if desired by adding one radiolabeled base triphosphate to the reaction mixture along with the unlabeled triphosphates at the beginning of the reaction. This produces multimer and product oligomers that are radiolabeled internally. For example, spiking the reaction mixture with $\alpha$-$^{32}$P-dCTP will produce oligomers internally labeled with $^{32}$P at every C residue. Alternatively, a radiolabeled primer oligomer can be used, which results in a 5' radiolabeled multimer.

Preferred polymerase enzymes that effectuate the synthesis of a multimer in rolling circle synthesis have high fidelity, high processivity, accept single-stranded templates, and have relatively low exonuclease activity. For DNA polymerization, i.e., formation of DNA multimers, suitable enzymes include, but are not limited to, DNA Polymerase I, Klenow fragment of DNA Polymerase I, T7 DNA Polymerase (exonuclease-free), T4 DNA Polymerase, Taq Polymerase, and AMV (or MuLV) Reverse Transcriptase or closely homologous mutants. This group of enzymes is also preferred. More preferably, the enzyme for DNA polymerization is the Klenow enzyme. For RNA polymerization, i.e., formation of RNA multimers, suitable enzymes include, but are not limited to, the phage polymerases and RNA Polymerase II. Preferred enzymes for RNA polymerization are T7, T4, and SP6 RNA Polymerases, as well as RNA Polymerase II and RNA Polymerase III or closely homologous mutants.

Useable nucleotide triphosphates are any that are used in standard PCR or polymerase technology. That is, any nucleotide triphosphate can be used in the rolling circle method that is capable of being polymerized by a polymerase enzyme. These can be both naturally occurring and synthetic nucleotide triphosphates. They include, but are not limited to, ATP, dATP, CTP, dCTP, GTP, dGTP, UTP, TTP, dUTP, 5-methyl-CTP, 5-methyl-dCTP, ITP, dITP, 2-amino-adenosine-TP, 2-amino-deoxyadenosine-TP, 2-thiothymidine triphosphate, pyrrolo-pyrimidine triphosphate, 2-thiocytidine as well as the alphathiotriphosphates for all of the above, and 2'-O-methyl-ribonucleotide triphosphates for all the above bases. Preferably, the nucleotide triphosphates are selected from the group consisting of dATP, dCTP, dGTP, TTP, and mixtures thereof. Modified bases can also be used in the method of the invention including, but not limited to, 5-Br-UTP, 5-Br-dUTP, 5-F-UTP, 5-F-dUTP, 5-propynyl dCTP, and 5-propynyl-dUTP. Most of these nucleotide triphosphates are widely available from commercial sources such as Sigma Chemical Co., St. Louis, Mo. Nucleotide triphosphates are advantageously used in the method of the present invention at least because they are generally cheaper than the nucleotide precursors used in machine synthesis. This is because the nucleotide triphosphates used herein are synthesized in as little as one step from natural precursors.

The rolling circle method can also be used to produce double-stranded DNA molecules. This is carried out by one of a number of methods. Rolling circle synthesis can be carried out separately on each of the complementary strands, and the multimer products combined at the end of the synthesis and then cleaved to give the desired duplex oligomers. Alternatively, two complementary single-stranded circular templates can be place in the reaction mixture simultaneously along with one primer for each strand where the primers are not complementary to each other. In this way, the two primer circular templates are formed and rolling circle synthesis can be carried out for both the complementary strands at the same time. This is possible because the two circular templates, although complementary to each other in sequence, cannot hybridize completely with each other as they are topologically constrained. As the complementary mulitmeric strands are formed, they combine to form the desired double-stranded multimer.

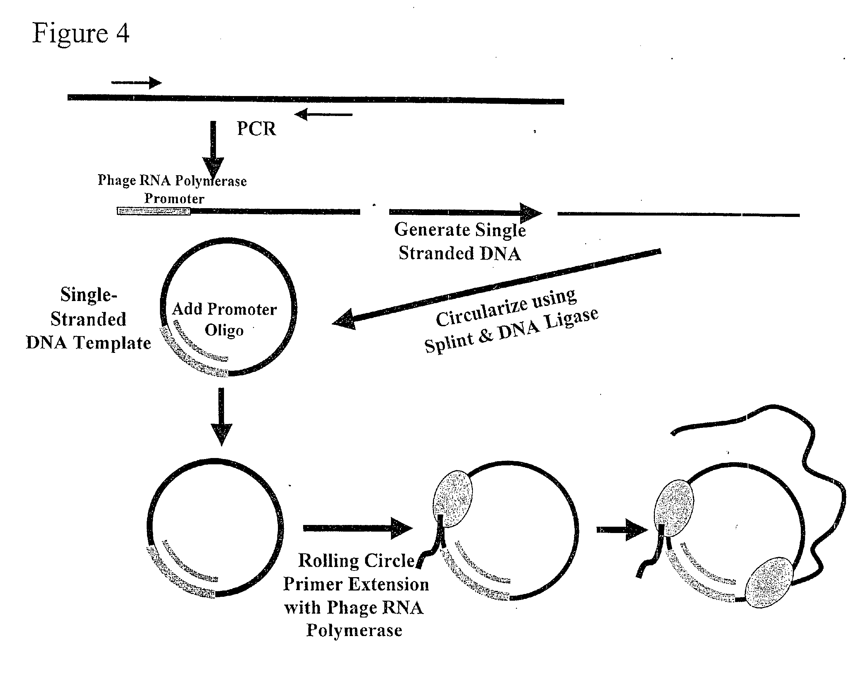

Perhaps the most efficient method for generating double-stranded DNA molecules is by simply adding a second primer that is complementary to the first RCA product (see, e.g. US5854033 and WO 9918241). Once the first multimeric product is formed, the second complementary primer can hybridize to it and serve as a template for synthesis of the second strand (see Figure 4).

The products generated from the synthetic method include linear or circular, single or double stranded DNA or RNA or analog multimer. The multimer can contain from about 60 to about $5 \times 10^6$ nucleotides, preferably about 500-100,000, or about 5-100,000 copies of the desired nucleotide sequences. Once formed, a linear multimer containing multiple copies of the desired sequence can be cleaved into single copy oligomers having the desired sequence either while synthesis is occurring or after oligonucleotide synthesis is complete.

UNSTRUCTURED NUCLEIC ACIDS (UNA)

In a preferred embodiment of the present invention, nucleic acid molecules having reduced levels of secondary structure are enzymatically synthesized for nanopore sequencing. Preferably, the synthesis uses a circular template to produce unstructured nucleic acid molecules with reduced secondary structure and with tandemly repeated sequences complementary to the template. Therefore, UNAs can be enzymatically synthesized for nanopore sequencing according to the teachings of Sampson (supra) and Baldarelli (supra) to reduce secondary structure in the molecule to be sequenced.

In another preferred embodiment, rolling circle amplification is used to generate UNAs. The continuous strand displacement property of the polymerase as it proceeds around the circular template is likely to be more efficient at displacing the nascent UNA strand than that expected for multiple cycle linear amplification methods such as asymmetric PCR. Importantly, UNAs can enable nanopore sequencing by reducing target intramolecular structures which can stall or prevent the target molecule from traversing the pore. Thus, UNAs synthesized by the rolling circle amplification method should be a superior method for generating targets for nanopore sequencing and greatly enable this technology.

The enzymatic synthesis of nucleic acids having modified nucleotides to reduce the levels of secondary structure (UNA) is described by Sampson (U.S.S.N. 09/358,141). Briefly summarized, Sampson teaches the synthesis of UNA by enzymatically incorporating nucleotide precursors which have a reduced ability (or no ability) to form base pairs with a complement which is also incorporated into the UNA. The nucleotides in the UNA must be capable of forming a base pair with a different yet still complementary nucleotide, which is preferably not in the UNA. This is due to the template-dependent polymerization of UNAs by enzymes. Therefore, a nucleotide precursor which is unable to form a stable base pair with a complement in the template will not be enzymatically incorporated into a nascent UNA polymer.

The base pairing concepts of UNAs are schematically depicted by the following formulas where A'≠ T' and G' ≠ C' represent disallowed base-pairing schemes, with the symbol ≠ representing the inability to form a base pair. [A*, T*, G*, and C*] represent a second group of bases capable of forming base pairs with A', T', G' and C' according to the general Watson-Crick base pair scheme of A=T and G=C, where = represents the ability to form a base pair. The same base pairing rules apply for RNA where U replaces T. (The horizontal base pairing symbols are not meant to represent the number of hydrogen bonds present in the base pair, but are meant only to indicate a stable base pair or lack of a stable base pair.)

$$(A'\neq T'; \ G'\neq C') \qquad\qquad (1)$$

$$(A'=T^*; \ T'=A^*; \ G'=C^*; \ C'=G^*) \qquad\qquad (2)$$

Formula 1 indicates that base pair analogs A'/T' and G'/C' are unable to form a stable base pair. However, as indicated in Formula 2, the bases of nucleotides A' T' G' and C' are capable of forming stable base pairs with a second group of nucleotide bases (A* T* G* C*).

UNAs may contain a mixture of nucleotide analogs and naturally-occurring nucleotides. UNAs of the present invention may also contain only nucleotide base analogs. More specifically, in accordance with the base pairing formulas outlined in Formula 1 and 2, nucleotides of the first group (A', T', G', C') and nucleotides of the second group (A*, T*, G*, and C*) may include combinations of natural bases and modified bases or include all modified bases. For example, A' and T', which does not form a stable base pair, may be comprised of one nucleotide base analog (A') and one natural nucleotide (T'). Alternatively, A' and T' may be comprised of two nucleotide base analogs. Nucleotide pairs from the second group (e.g. A* and T*) may or may not form stable base pairs (A*=T* or A*≠T*).

UNAs may contain both A'/T' base pair analogs that do not form stable base pairs and G/C base pairs that do form stable base pairs. Alternatively, UNAs may contain G'/C' base pair analogs that do not form stable base pairs and A/T base pairs that do form stable base pairs. UNAs may also contain both sets of analogs that do not form stable base pairs (A'≠T' and G'≠C'). For the present invention, nucleotide from the first and second class (e.g. A', A*) may be mixed in the same molecule. However, it is preferred that a single UNA molecule possess no more than one of each type of nucleotide (e.g. only A' T' G and C) which results in only one type of base-pairing scheme for each potential base-pair.

Polymerization methodologies that utilize template dependent DNA or RNA polymerases are preferred methods for copying genetic material of unknown sequence from biological sources for subsequent sequence and expression analyses. Thus UNAs, which are produced preferably by enzymatic methods, are well suited for generating oligonucleotides and polynucleotides for subsequent nanopore sequencing. Moreover, since preferred UNAs are synthesized using DNA and RNA polymerases, UNAs may be synthesized having lengths ranging from several nucleotides to several thousand nucleotides.

Any enzyme capable of incorporating naturally-occurring nucleotides, nucleotides base analogs, or combinations thereof into a polynucleotide may be utilized in accordance with the present invention. As examples without limitation, the enzyme can be a primer/DNA template dependent DNA polymerase, a primer/RNA template dependent reverse transcriptase or a promoter-dependent RNA polymerase. Non-limiting examples of DNA polymerases include *E. coli* DNA polymerase I, *E. coli* DNA polymerase I Large Fragment (Klenow fragment), or phage T7 DNA polymerase. The polymerase can be a thermophilic polymerase such as *Thermus aquaticus* (Taq) DNA polymerase, *Thermus flavus* (Tfl) DNA polymerase, *Thermus Thermophilus* (Tth) Dna polymerase, *Thermococcus litoralis* (Tli) DNA polymerase, *Pyrococcus furiosus* (Pfu) DNA polymerase, Vent™ DNA polymerase, or *Bacillus stearothermophilus* (Bst) DNA polymerase. Non-limiting examples of reverse transcriptases include AMV Reverse Transcriptase, MMLV Reverse Transcriptase and HIV-1 reverse transcriptase. Non-limiting examples of RNA polymerases suitable for generating RNA version of UNAs include the bacteriophage RNA polymerases from SP6, T7 and T3. Furthermore, any molecule capable of using a DNA or an RNA molecule as a template to synthesize another DNA or RNA molecule can be used in accordance with the present invention. (e.g. self-replicating RNA).

Primer/DNA template-dependent DNA polymerases, primer/RNA template-dependent reverse transcriptases and promoter-dependent RNA polymerases incorporate nucleotide triphosphates into the growing polynucleotide chain according to the standard Watson and Crick base-pairing interactions (see for example; Johnson, Annual Review in Biochemistry, 62; 685-713 (1993), Goodman et al., Critical Review in Biochemistry and Molecular Biology, 28; 83-126 (1993) and Chamberlin and Ryan, The Enzymes, ed. Boyer, Academic Press, New York, (1982) pp 87-108). Some primer/DNA template dependent DNA polymerases and primer/RNA template dependent reverse transcriptases are capable of incorporating non-naturally occurring triphosphates into polynucleotide chains when the correct complementary nucleotide is present in the template sequence. For example, Klenow fragment and AMV reverse transcriptase are capable of incorporating the base analogue iso-guanosine opposite iso-cytidine residues in the template sequence (Switzer et al., Biochemistry 32; 10489-10496 (1993). Similarly, Klenow fragment and HIV-1 reverse transcriptase are capable of incorporating the base analogue 2,4-diaminopyrimidine opposite xanthosine in a template sequence (Lutz et al., Nucleic Acids Research 24; 1308-1313 (1996)).

UNAs can also be generated using a polymerase extension reaction followed by a strand-selective exonuclease digestion (Little et al., J. Biol Chem. 242, 672 (1967) and Higuchi and Ochamn, Nucleic Acids Research, 17; 5865- (1989)). For example, a target-specific primer is extended in an isothermal reaction using a DNA polymerase or reverse transcriptase in the presence of the appropriate UNA nucleotide triphosphates and a 5'-phosphorylated DNA template. The DNA template strand of the resulting duplex is then specifically degraded using the 5'-phosphorly-specific lambda exonuclease. A kit for performing the latter step is the Strandase Kit™ currently marketed by Novagen (Madison,WI).

Single-stranded ribonucleotide (RNA) versions of UNAs can be synthesized using *in vitro* transcription methods which utilize phage promoter-specific RNA polymerases such as SP6 RNA polymerase, T7 RNA polymerase and T3 RNA polymerase (see for example Chamberlin and Ryan, The Enzymes, ed. Boyer, Qacademic Press, New York, (1982) pp87-108 and Melton et al., Nucleic Acids Research, 12; 7035 (1984)). For these methods, a double stranded DNA corresponding to the target sequence is generated using PCR methods known in the art in which a phage promoter sequence is incorporated upstream of the target sequence. This double-stranded DNA is then used as the template in an *in vitro* transcription reaction containing the appropriate phage polymerase and the ribonucleotide triphosphate UNA analogues. Alternatively, a single stranded DNA template prepared according to the method of Milligan and Uhlenbeck, (Methods in Enzymology, 180A, 51-62 (1989)) can be used to generate RNA versions of UNAs having any sequence. A benefit of these types of *in vitro* transcription methods is that they can result in a 100 to 500 fold amplification of the template sequence.

## Structural Modifications to Nucleotides

Nucleotide base analogues having fewer structural changes can also be efficient substrates for DNA polymerase reactions. For example, a number of polymerases can specifically incorporate inosine across cytidine residues (Mizusawa et al., Nucleic Acids Research, 14; 1319 (1986). The analogue 2-aminoadenosine triphosphate can also be efficiently incorporated by a number of DNA polymerases and reverse transcriptases (Bailly and Waring, Nucleic Acids Research, 23; 885 (1996). In fact, 2-aminoadenosine is a natural substitute for adenosine in S-2L cyanophage genomic DNA. However, for the present invention 2-aminoadenosine is defined as a non-naturally occurring base. The 2-aminoadenosine ribonucleotide-5'-triphosphate is a good substrate *for E. coli* RNA polymerase (Rackwitz and Scheit, Eur. J. Biochem., 72, 191 (1977)). The adenosine analogue 2-aminopurine can also be efficiently incorporated opposite T residues by *E. coli* DNA polymerase (Bloom et al., Biochemistry 32; 11247-11258 (1993) but can mispair with cytidine residues as well (see Law et al., Biochemistry 35; 12329-12337 (1996)).

Any structural modifications to a nucleotide that do not inhibit the ability of an enzyme to incorporate the nucleotide analogue may be used in the present invention if the modifications do not result in a violation of the base pairing rules set forth in the present invention. Modifications include but are not limited to structural changes to the base moiety (e.g. C5-bromouridine, C5-fluorouridine, C5-iodouridine, C5-propynyl-uridine, C5-propynyl-cytidine, C5-methylcytidine, 2-aminoadenosine, 7-deazaadenosine, 7-deazaguanosine, 8-oxoadenosine, 8-oxoguanosine), changes to the ribose ring (e.g. 2'-hydroxyl, 2'-fluro), and changes to the phosphodiester linkage (e.g. phosphorothioates and 5' -N-phosphoamidite linkages).

Watson-Crick base-pairing schemes can accommodate a number of modifications to the ribose ring, the phosphate backbone and the nucleotide bases (Saenger, Principles of Nucleic Acid Structure, Springer-Verlag, New York, NY. 1983). Certain modified bases such as inosine, 7-deazaadenosine, 7-deazaguanosine and deoxyuridine decrease the stability of base-pairing interactions when incorporated into polynucleotides. The dNTP forms of these modified nucleotides are efficient substrates for DNA polymerases and have been used to reduce sequencing artifacts that result from target and extension product secondary structures (Mizusawa et al., Nucleic Acids Research, 14; 1319. 1986). Other modified nucleotides, such as 5-methylcytidine, C-5 propynyl-cytidine, C-5 propynyl-uridine and 2-aminoadenosine increase the stability of duplex when incorporated into polynucleotides (Wagner et al., Science, 260; 1510. 1993) and have been used to increase the hybridization efficiency between oligonucleotide probes and target sequences.

## Selection of Nucleotides for UNAs

In accordance with the present invention, UNAs are produced such that regions of self-complementarity in a UNA have a reduced ability to form stable hybrids with each other. Therefore, UNAs have a reduced level of duplex or higher order secondary structure under conditions permitting duplex formation in naturally occurring DNA of similar size. Complementary nucleotides for producing UNAs are selected such that a first nucleotide base is not capable of forming a stable base pair with a nucleotide complement. The two complementary nucleotides may have one naturally-occurring base and one base analog or may have two base analogs. The complementary nucleotides that are unable to form a stable base pair are used to produce UNA with reduce the levels of intramolecular base pairing by reducing hybridization between sequence elements within the UNA that are substantially complementary. Complementary nucleotides that are unable to form stable pairs may also be used in sequences of the UNA that do not have substantially self-complementary sequences within the same UNA polynucleotide molecule.

In addition, it is preferable that the complementary nucleotides in a UNA that are unable to form stable base pairs, are capable of forming stable base pairs with at least one nucleotide complement present in a second polynucleotide molecule such as a template. Preferably, the second polynucleotide molecule contains sequences elements substantially complementary to sequence elements in the UNA to allow hybridization of part or all of the second polynucleotide to the UNA. Complementary sequence elements of the second polynucleotide may contain naturally-occurring bases or base analogs.

**2-Aminoadenosine (D), 2-Thiothymidine (2-thioT), Inosine (I) and Pyrrolo-pyrimidine (P)**

In a particularly preferred embodiment, the nucleotide analogs 2-aminoadenosine (D), 2-thiothymidine (2-thioT), inosine (I) and pyrrolo-pyrimidine (P) are used to generate nucleic acid molecules that are unable to form stable secondary structures yet retain their ability to form Watson-Crick base-pairs with oligonucleotides composed of the four natural bases. The structures of the D/2-thioT, I/P and the four natural base pairs along with various combinations of the natural and base analogs are shown in Figure 8.

Naturally occurring Watson-Crick base-pairing is defined by specific hydrogen bonding interactions between the bases of adenine and thymine (or uracil) and between guanine and cytosine. Positioning of hydrogen-bond donors (e.g. amino groups) and hydrogen-bond acceptors (e.g. carbonyl groups) on purine and pyrimidine bases place structural constraints on the ability of two nucleoside bases to form stable hydrogen bonds. Figure 8 shows the structures of the bases and the relative orientations of the bases to each other in a Watson-Crick base pair. In addition, an inosine:cytosine base pair is shown. The inosine-cytosine base pair is identical to a G-C base pair except that the I-C base pair lacks the hydrogen bond donor of the 2-amino group of guanine which is missing in inosine.

**2-Aminoadenosine (D), 2-Thiothymidine (2-thioT)**

Without being limited by theory, a D/2-thioT base pair analog is prevented from forming a stable base pair presumably due to a steric clash between the thio group of 2-thioT and the exocyclic amino group of 2-aminoadenosine as a result of the larger atomic radius of the sulfur atom. This tilts the nucleotide bases relative to one another such that only one hydrogen bond is able to form. It is also known that thionyl sulfur atoms are poorer hydrogen-bonding acceptors than carbonyl oxygen atoms which could also contribute to the weakening of the D/2-thioT base pair.

Furthermore, the 2-aminoadenosine (D) is capable of forming a stable base-pair with thymidine (T) through three hydrogen bonds in which a third hydrogen bonding interaction is formed between the 2-amino group and the C2 carbonyl group of thymine. As a result, the D/T base pair is more stable thermodynamically than an A/T base pair. In addition, 2-thiothymidine (2-thioT) is capable of forming a stable hydrogen bonded base pair with adenosine (A) which lacks an exocyclic C2 group to clash with the 2-thio group.

Therefore, polynucleotide molecules with 2-aminoadenosine (D) and 2-thioT replacing A and T respectively are unable to form intramolecular D/2-thioT base pairs but are still capable of hybridizing to polynucleotides of substantially complementary sequence comprising A and T and lacking D and 2-thioT. Without being limited by theory, the aforementioned proposed mechanisms regarding the factors responsible for stabilizing and disrupting the A/T and G/C analogue pairs are not meant in anyway to limit the scope of the present invention and are valid irrespective of the nature of the specific mechanisms.

Gamper and coworkers (Kutyavin et al. Biochemistry, 35; 11170 (1996)) determined experimentally that short oligonucleotide duplexes containing D/T base pairs that replace A/T base pairs have melting temperatures (Tm) as much as 10° C higher than duplexes of identical sequence composed of the four natural nucleotides. This is due mainly to the extra hydrogen bond provide by the 2-amino group. However, the duplexes designed to form opposing D/2-thioT base-pairs exhibited Tms as much as 25° C lower than the duplex of identical sequence composed of standard A/T base-pairs. The authors speculate that this is mainly due to the steric clash between the 2-thio group and the 2-amino group which destabilizes the duplex. Deoxyribonucleotides in this study were synthesized using chemical methods.

Although the base-pairing selectivity for these analog pairs has been experimentally tested for only DNA duplexes, it is likely that these same rules will hold for RNA duplexes and DNA/RNA heteroduplexes as well. This would allow for RNA versions of UNAs to be generated by transcription of PCR or cDNA products using the ribonucleotide triphosphate forms of the UNA analog pairs and RNA polymerases.

**Inosine (I) and Pyrrolo-pyrimidine (P)**

The inosine (I) and pyrrolo-pyrimidine (P) I/P base pair analog is also depicted in Figure 8. Inosine, which lacks the exocyclic 2-amino group of guanine, forms a stable base pair with cytosine through two hydrogen bonds (vs. three for G/C). The other member of the I/P analog is pyrrolo-pyrimidine (P) which is capable of forming a stable base pair with guanine despite the loss of the 4-amino hydrogen bond donor of cytosine. Figure 8 shows that a P/G base pair is also formed through two hydrogen bonds. The N7 group of P is spatially confined by the pyrrole ring and is unable to form a hydrogen bond with the C6 carbonyl O of guanine. However, this does not prevent the formation of the other two hydrogen bonds between P/G. The I/P base pair is only capable of forming one hydrogen bond (as depicted in Figure 8) and is therefore not a stable base pair. As a result, polynucleotide molecules with I and P replacing G and C respectively are unable to form intramolecular I/P base pairs but are still capable of hybridizing to polynucleotides of substantially complementary sequence comprising G and C and lacking I and P.

Woo and co-workers (Woo et al., Nucleic Acids Research, 24; 2470 (1996)) showed that introducing either P or I into 28-mer duplexes to form P/G and I/C base-pairs decreased the Tm of the duplex by -0.5 and -1.9° C respectively per modified base-pair. These values reflect the slight destabilization attributable to the G/P pair and a larger destabilization due to the I/C pair. However, introducing P and I into the duplexes such that opposing I/P base-pairs are formed reduced the Tm by -3.3° C per modified base-pair. Therefore the I/P base pairs are more destabilizing.

## UNAs comprising D, 2-thioT, I, and P

In accordance with the present invention, nucleic acid molecules with reduced secondary structure (UNAs) are generated by performing primer dependent, template directed polymerase reactions using the nucleotide 5'-triphosphate forms of the appropriate analog pairs. These include; 2-amino-2'-deoxyadenosine-5'-triphosphate (dDTP), 2-thiothymidine-5'-triphosphate (2-thioTTP), 2'-deoxyinosine-5'-triphosphate (dITP) and 2'-deoxypyrrolo-pyrimidine-5'-triphosphate (dPTP). For example, a reaction containing dDTP, 2-thioTTP, dCTP and dGTP will generate UNAs which are unable to form intramolecular A/T base pairs. Likewise, a reaction containing dATP, dTTP, dPTP and dITP will generate UNAs which are unable to form intramolecular P/I (modification of G/C) base pairs. A polymerization reaction containing both analog pairs, dDTP, 2-thioTTP; and dPTP, dITP will generate UNAs that have no predicted intramolecular base-pairing interactions. However, since 2-aminoadenosine, 2-thiothymidine, pyrrolo-pyrimidine, and inosine are still capable of forming stable base pairs with thymidine, adenosine, cytidine and guanosine respectively, all three types of UNAs should be able to specifically hybridize intermolecularly to oligonucleotides composed of the four natural bases.

In yet another preferred embodiment, it is recognized that UNAs of the present invention may contain various levels of secondary structure. For example, UNAs may contain only G/C intramolecular base pairs and not A/T intramolecular base pairs. Alternatively, UNAs may contain only A/T intramolecular base pairs and not G/C intramolecular base pairs. UNAs potentially containing only G/C intramolecular base pairs are generated by enzymatically incorporating the triphosphate forms of 2-aminoadenosine, 2-thiothymidine, guanosine, and cytosine into a polynucleotide. The resulting UNA polynucleotide is not capable of forming intramolecular A/T base pairs, but is still capable of forming intramolecular G/C base pairs. The aforementioned mechanisms which may account for the observed disruption of the A/T and G/C analogue pairs is not meant in anyway to limit the scope of the present invention and is valid irrespective of the nature of the specific mechanisms.

## UNAs comprising D, 2-thioT, 2-thioC, and G

In yet another preferred embodiment of the present invention, the nucleotide base pair analogs D/2-thiothymidine and 2-thiocytosine/guanosine (2-thioC/G) are used in primer dependent polymerase reactions to generate nucleic acid molecules that are unable to form stable secondary structures yet retain their ability to form Watson-Crick base pairs with oligonucleotides composed of the four natural bases. 2-thioC and G are unable to form a stable base pair. The presence of a 2-thiol exocyclic group in cytosine replacing the C2 carbonyl group effectively removes the hydrogen bond acceptor at that position and causes a steric clash due to the large ionic radius of sulfur as compared to oxygen. As a result, 2-thioC/G is only capable of forming a single hydrogen bond and is thus not a stable base pair. However, 2-thioC and I are capable of forming a stable base pair through two hydrogen bonds since the removal of the 2-amino exocyclic group of guanine that results in inosine effectively removes the steric clash between the C2 sulfur of 2-thioC and the 2-amino group of guanine.

Therefore, polynucleotide molecules with reduced secondary structure are generated enzymatically using the 5'-triphosphate forms of the base pair analogs. These include; 2-amino-2'-deoxyadenosine-5'-triphosphate (dDTP), 2-thiothymidine-5'-triphosphate (2-thioTTP), 2'-deoxyguanosine-5'-triphosphate (dGTP) and 2-thio-2'-deoxycytidine-5'-triphosphate (2-thio-dCTP). For example, a reaction with 2-thio-dCTP, dGTP, dATP, dTTP will generate UNAs that can form only A/T base pairs. A polymerization reaction containing both analog pairs, 2-thio-dCTP/dGTP, and dDTP/2-thioTTP will generate UNAs that have no predicted intramolecular base-pairing interactions. However, since 2-aminoadenosine, 2-thiothymidine, 2-thiocytidine and guanosine are still capable of forming stable base pairs with thymidine, adenosine, inosine and cytidine respectively, UNAs comprising (A, T, 2-thioC, G) or (D, 2-thioT, 2-thioC, G) should be able to specifically hybridize to oligonucleotides composed of the appropriate bases according to the base pairing rules discussed.

The 2-thioC/G base pair analog provides an example of a base pair analog comprising a natural nucleotide base and a nucleotide base analog which can not form a stable base pair. As previously stated, polynucleotides containing 2-thiocytidine and guanosine cannot form intramolecular 2-thioC/G base pairs. However, these polynucleotides can form base pairs with polynucleotides of substantially complementary sequences through 2-thioC/I and C/G base pairs. Therefore, UNAs comprising 2-thioC/G are capable of hybridizing to polynucleotide molecules also containing base analogs (inosine).

NANOPORE SEQUENCING

In another preferred embodiment, nucleic acid molecules having tandemly repeated sequences are sequenced by nanopore sequencing. The tandemly repeated sequences may be synthesized enzymatically or chemically by any method desired by one skilled in the art. It is particularly preferred that nucleic acid molecules having tandem repeats are synthesized by rolling circle amplification as described above.

In another preferred embodiment, nucleic acid molecules having reduced levels of secondary structure (UNAs) are sequenced by nanopore sequencing. UNAs may be chemically synthesized or enzymatically synthesized as described above. In a particularly preferred embodiment, UNAs having tandem repeats are synthesized enzymatically using rolling circle amplification for nanopore sequencing.

In general, nanopore sequencing is used to evaluate a polymer molecule which includes linearly connected (sequential) monomer residues and is described by Baldarelli et al. (USP 6,015,714). In accordance with the present invention, preferred polymers are nucleic acids and the monomers are nucleotides. Nanopore sequencing involves the use of two separate pools of a medium and an interface between the pools. The interface between the pools is capable of interacting sequentially with the individual monomer residues of a single polymer present in one of the pools. Interface dependent measurements are continued over time, as individual monomer residues of a single polymer interact sequentially with the interface, yielding data suitable to infer a monomer-dependent characteristic of the polymer. Several individual polymers, e.g., in a heterogeneous mixture, can be characterized or evaluated in rapid succession, one polymer at a time, leading to characterization of the polymers in the mixture.

The monomer-dependent characterization achieved by nanopore sequencing may include identifying physical characteristics such as the number and composition of monomers that make up each individual molecule, preferably in sequential order from any starting point within the nucleic acid or its beginning or end. A heterogeneous population of nucleic acids may be characterized, providing a distribution of characteristics (such as size) within the population. Where the monomers within a given nucleic acid molecule are heterogeneous, the method can be used to determine their sequence.

The interface between the pools is designed to allow passage of the monomers of one nucleic acid molecule at a time. As described in greater detail below, the useful portion of the interface may be a passage in or through an otherwise impermeable barrier, or it may be an interface between immiscible liquids.

The medium used in nanopore sequencing may be any fluid that permits adequate nucleic acid mobility for interface interaction. Typically, the medium will be liquids, usually aqueous solutions or other liquids or solutions in which the nucleic acids can be distributed. When an electrically conductive medium is used, it can be any medium which is able to carry electrical current. Such solutions generally contain ions as the current conducting agents, e.g., sodium, potassium, chloride, calcium, cesium, barium, sulfate, or phosphate. Conductance across the pore or channel is determined by measuring the flow of current across the pore or channel via the conducting medium. A voltage difference can be imposed across the barrier between the pools by conventional means. Alternatively, an electrochemical gradient may be established by a difference in the ionic composition of the two pools of medium, either with different ions in each pool, or different concentrations of at least one of the ions in the solutions or media of the pools. In this embodiment of the invention, conductance changes are measured and are indicative of monomer-dependent characteristics.

The term "ion permeable passages" used in this embodiment of the invention includes ion channels, ion-permeable pores, and other ion-permeable passages, and all are used herein to include any local site of transport through an otherwise impermeable barrier. For example, the term includes naturally occurring, recombinant, or mutant proteins which permit the passage of ions under conditions where ions are present in the medium contacting the channel or pore. Synthetic pores are also included in the definition. Examples of such pores can include, but are not limited to, chemical pores formed, e.g., by nystatin, ionophores, or mechanical perforations of a membranous material. Proteinaceous ion channels can be voltage-gated or voltage independent, including mechanically gated channels (e.g., stretch-activated $K^+$ channels), or recombinantly engineered or mutated voltage dependent channels (e.g., $Na^+$ or $K^+$ channels constructed as is known in the art).

Another type of channel is a protein which includes a portion of a bacteriophage receptor which is capable of binding all or part of a bacteriophage ligand (either a natural or functional ligand) and transporting bacteriophage DNA from one side of the interface to the other. The nucleic acid to be characterized includes a portion which acts as a specific ligand for the bacteriophage receptor, so that it may be injected across the barrier/interface from one pool to the other.

The protein channels or pores of the invention can include those translated from one or more natural and/or recombinant DNA molecule(s) which includes a first DNA which encodes a channel or pore forming protein and a second DNA which encodes a monomer-interacting portion of a monomer polymerizing agent (e.g., a nucleic acid polymerase or exonuclease). The expressed protein or proteins are capable of noncovalent association or covalent linkage (any linkage herein referred to as forming an "assemblage" of "heterologous units"), and when so associated or linked, the polymerizing portion of the protein structure is able to polymerize monomers from a template polymer, close enough to the channel forming portion of the protein structure to measurably affect ion conductance across the channel. Alternatively, assemblages can be formed from unlike molecules, e.g., a chemical pore linked to a protein polymerase; these assemblages fall under the definition of a "heterologous" assemblage.

Nanopore sequencing also includes the use of recombinant fusion protein(s) translated from the recombinant DNA molecule(s) described above, so that a fusion protein is formed which includes a channel forming protein linked as described above to a monomer-interacting portion of a nucleic acid polymerase. Preferably, the nucleic acid polymerase portion of the recombinant fusion protein is capable of catalyzing polymerization of nucleotides. Preferably, the nucleic acid polymerase is a DNA or RNA polymerase, more preferably T7 RNA polymerase.

The nucleic acid being characterized may remain in its original pool, or it may cross the passage. Either way, as a given nucleic acid molecule moves in relation to the passage, individual nucleotides interact sequentially with the elements of the interface to induce a change in the conductance of the passage. The passages can be traversed either by nucleic acid transport through the central opening of the passage so that the nucleic acid passes from one of the pools into the other, or by the nucleic acid traversing across the opening of the passage without crossing into the other pool. In the latter situation, the nucleic acid is close enough to the channel for its nucleotides to interact with the passage and bring about the conductance changes which are indicative of nucleic acid characteristics. The nucleic acid can be induced to interact with or traverse the pore, e.g., as described below, by a polymerase or other template-dependent nucleic acid replicating catalyst linked to the pore which draws the nucleic acid across the surface of the pore as it synthesizes a new nucleic acid from the template polymer, or by a polymerase in the opposite pool which pulls the nucleic acid through the passage as it synthesizes a new nucleic acid from the template polymer. In such an embodiment, the nucleic acid replicating catalyst is physically linked to the ion-permeable passage, and at least one of the conducting pools contains monomers suitable to be catalytically linked in the presence of the catalyst. A "polymer replicating catalyst," "polymerizing agent" or "polymerizing catalyst" is an agent that can catalytically assemble monomers into a nucleic acid in a template dependent fashion--i.e., in a manner that uses the nucleic acid molecule originally provided as a template for reproducing that molecule from a pool of suitable monomers. Such agents include, but are not limited to, nucleotide polymerases of any type, e.g., DNA polymerases, RNA polymerases, tRNA and ribosomes.

The characteristics of the nucleic acid can be identified by the amplitude or duration of individual conductance changes across the passage. Such changes can identify the monomers in sequence, as each monomer will have a characteristic conductance change signature. For instance, the volume, shape, or charges on each monomer will affect conductance in a characteristic way. Likewise, the size of the entire nucleic acid can be determined by observing the length of time (duration) that monomer-dependent conductance changes occur. Alternatively, the number of nucleotides in a nucleic acid (also a measure of size) can be determined as a function of the number of nucleotide-dependent conductance changes for a given nucleic acid traversing a passage. The number of nucleotides may not correspond exactly to the number of conductance changes, because there may be more than one conductance level change as each nucleotide of the nucleic acid passes sequentially through the channel. However, there will be a proportional relationship between the two values which can be determined by preparing a standard with a nucleic acid of known sequence.

The mixture of nucleic acids used in nanopore sequencing does not need to be homogenous. Even when the mixture is heterogeneous, only one molecule interacts with a passage at a time, yielding a size distribution of molecules in the mixture, and/or sequence data for multiple nucleic acid molecules in the mixture.

In other embodiments, the channel is a natural or recombinant bacterial porin molecule that is relatively insensitive to an applied voltage and does not gate. Preferred channels for use in the invention include the $\alpha$-hemolysin toxin from *S. aureus* and maltoporin channels.

In other preferred embodiments, the channel is a natural or recombinant voltage-sensitive or voltage gated ion channel, preferably one which does not inactivate (whether naturally or through recombinant engineering as is known in the art). "Voltage sensitive" or "gated" indicates that the channel displays activation and/or inactivation properties when exposed to a particular range of voltages.

In an alternative embodiment, the pools of medium are not necessarily conductive, but are of different compositions so that the liquid of one pool is not miscible in the liquid of the other pool, and the interface is the immiscible surface between the pools. In order to measure the characteristics of the nucleic acid, a nucleic acid molecule is drawn through the interface of the liquids, resulting in an interaction between each sequential nucleotide of the nucleic acid and the interface. The sequence of interactions as the nucleotide of the nucleic acid are drawn through the interface is measured, yielding information about the sequence of nucleotides that characterize the polymer. The measurement of the interactions can be by a detector that measures the deflection of the interface (caused by each nucleotide passing through the interface) using reflected or refracted light, or a sensitive gauge capable of measuring intermolecular forces. Several methods are available for measurement of forces between macromolecules and interfacial assemblies, including the surface forces apparatus (Israelachvili, Intermolecular and Surface Forces, Academic Press, New York, 1992), optical tweezers (Ashkin et al., Oppt. Lett., 11: 288, 1986; Kuo and Sheetz, Science, 260: 232, 1993; Svoboda et al., Nature 365: 721, 1993), and atomic force microscopy (Quate, F. Surf. Sci. 299: 980, 1994; Mate et al., Phys. Rev. Lett. 59: 1942, 1987; Frisbie et al., Science 265: 71, 1994).

The interactions between the interface and the nucleotides in the nucleic acid are suitable to identify the size of the nucleic acid molecule, e.g., by measuring the length of time during which the nucleic acid interacts with the interface as it is drawn across the interface at a known rate, or by measuring some feature of the interaction (such as deflection of the interface, as described above) as each nucleotide of the nucleic acid is sequentially drawn across the interface. The interactions can also be sufficient to ascertain the identity of individual nucleotides in the polymer.

Nanopore sequencing is capable of sequencing double stranded or single stranded nucleic acids, by (1) providing two separate, adjacent pools of a medium and an interface (e.g., a lipid bilayer) between the two pools, the interface having a channel (e.g., bacterial porin molecules) so dimensioned as to allow sequential monomer-by-monomer passage from one pool to another of only one nucleic acid nucleic acid at a time; (2) placing the nucleic acid nucleic acid to be sequenced in one of the two pools; and (3) taking measurements (e.g., ionic flow measurements, including measuring duration or amplitude of ionic flow blockage) as each of the nucleotide monomers of the nucleic acid nucleic acid passes through the channel, so as to sequence the nucleic acid polymer. The interface can include more than one channel in this method. In some cases, the nucleic acid nucleic acid can interact with in inner surface of the channel. The sequencing of a nucleic acid, as used herein, is ot limited to identifying specific nucleotide monomers, but can include distinguishing one type of monomer from another type of monomer (e.g., purines from pyrimidines).

The two pools can contain an electrically conductive medium (e.g., an aqueous solution), in which case a voltage can be optionally applied across the interface to facilitate movement of the nucleic acid nucleic acid through the channel and the taking of measurements. Such measurements are interface-dependent, i.e., the measurements are spatially or temporally related to the interface. For example, ionic measurements can be taken when the nucleic acid traverses an internal limiting (in size or conductance) aperture of the channel. In this case, the flow of ions through the channel, and especially through the limiting aperture of the channel, is affected by the size or charge of the nucleic acid and the inside surface of the channel. These measurements are spatially related to the interface because one measures the ionic flow through the interface as specific monomers pass a specific portion (the limiting aperture) of the interface channel.

To maximize the signal to noise ratio when ionic flow measurements are taken, the interface surface area facing a chamber is preferably less than 0.02 mm$^2$. In general, the interface containing the channels should have a design which minimizes the total access resistance to less than 20% of the theoretical (calculated) minimal convergence resistance. The total access resistance is the sum of the resistance contributed by the electrode/electrolyte interface, salt bridges, and the medium in the channel. The resistance of the medium in the channel includes the bulk resistance, the convergence resistance at each end of the channel, and the intra-channel resistance.

In addition, measurements can be temporally related to the interface, such as when a measurement is taken at a pre-determined time or range of times before or after each monomer passes into or out of the channel.

As an alternative to voltage, a nucleic acid polymerase or exonuclease can be provided in one of the chambers to draw the nucleic acid nucleic acid through the channel as discussed below.

Nanopore sequencing offers advantages in nucleotide sequencing, e.g., reduced number of sequencing steps, higher speed of sequencing, and increased length of the nucleic acid to be sequenced. The speed of the method and the size of the polymers it can sequence are particular advantages of the invention. The linear nucleic acid may be very large, and this advantage will be especially useful in reducing template preparation time, sequencing errors and analysis time currently needed to piece together small overlapping fragments of a large gene or stretch of polymer.

In one embodiment, nanopore sequencing involves measurements of ionic current modulation as the monomers (e.g., nucleotides) of a linear nucleic acid (e.g., nucleic acid molecule) pass through or across a channel in an artificial membrane. During nucleic acid passage through or across the channel, ionic currents are reduced in a manner that reflects the properties of the nucleic acid (length, concentration of polymers in solution, etc.) and the identities of the monomers. In the second embodiment, an immiscible interface is created between two immiscible liquids, and, as above, nucleic acid passage through the interface results in monomer interactions with the interface which are sufficient to identify characteristics of the nucleic acid and/or the identity of the monomers.

I. Polymer Analysis Using Conductance Changes Across An Interface

Sensitive single channel recording techniques (i.e., the patch clamp technique) can be used in the invention, as a rapid, high-resolution approach allowing differentiation of nucleotide bases of single DNA molecules, and thus a fast and efficient DNA sequencing technique or a method to determine nucleic acid size or concentration. Baldarelli et al. (supra) describe methods to orient DNA to a pore molecule in two general configurations and record conductance changes across the pore. One method is to use a pore molecule such as the receptor for bacteriophage lambda (LamB) or α-hemolysin, and to record the process of DNA injection or traversal through the channel pore when that channel has been isolated on a membrane patch or inserted into a synthetic lipid bilayer. Another method is to fuse a DNA polymerase molecule to a pore molecule and allow the polymerase to move DNA over the pore's opening while recording the conductance across the pore. A third method is to use a polymerase on the trans side of the membrane/pore divider to pull a single stranded nucleic acid through the pore from the cis side (making it double stranded) while recording conductance changes. A fourth method is to establish a voltage gradient across a membrane containing a channel (e.g., α-hemolysin) through which a single stranded or double stranded DNA is electrophoresed.

The apparatus used for this embodiment includes 1) an ion-conducting pore or channel, perhaps modified to include a linked or fused polymerizing agent, 2) the reagents necessary to construct and produce a linear nucleic acid to be characterized, or the polymerized molecule itself, and 3) an amplifier and recording mechanism to detect changes in conductance of ions across the pore as the nucleic acid traverses its opening.

A variety of electronic devices are available which are sensitive enough to perform the measurements used in the invention, and computer acquisition rates and storage capabilities are adequate for the rapid pace of sequence data accumulation.

A. Characteristics Identified by Nanopore sequencing

1) Size/Length of Molecules

The size or length of a nucleic acid can be determined by measuring its residence time in the pore or channel, e.g., by measuring duration of transient blockade of current. The relationship between this time period and the length of the nucleic acid can be described by a reproducible mathematical function which depends on the experimental condition used. The function is likely a linear function for a given type of nucleic acid (e.g., DNA, RNA, polypeptide), but if it is described by another function (e.g., sigmoidal or exponential), accurate size estimates may be made by first preparing a standard curve using known sizes of like linear molecules.

2) Identity of Residues/Monomers

The chemical composition of individual monomers is sufficiently variant to cause characteristic changes in channel conductance as each monomer traverses the pore due to physical configuration, size/volume, charge, interactions with the medium, etc. For example, our experimental data suggest that poly(C) RNA reduces conductance more than does poly(A) RNA, indicating a measurable physical difference between pyrimidines and purines that is one basis of nucleotide identification in this invention.

The nucleotide bases of DNA will influence pore conductance during traversal, but if the single channel recording techniques are not sensitive enough to detect differences between normal bases in DNA, it is practical to supplement the system's specificity by using modified bases. The modifications should be asymmetrical (on only one strand of double stranded template), to distinguish otherwise symmetrical base pairs.

Modified bases may be used in nanopore sequencing. These include: 1) methylated bases (lambda can package and inject DNA with or without methylated A's and C's), 2) highly modified bases found in the DNA of several bacteriophage (e.g. T4, SP15), many of which involve glycosylations coupled with other changes (Warren, 1980, Ann. Rev. Microbiol., 34: 137-58), and 3) the modified nucleotide triphosphates that can be incorporated by DNA polymerase (e.g. biotinylated, digoxigenated, and fluorescently tagged triphosphates).

Nanopore sequencing should avoid conditions that lead to secondary structure in the nucleic acid to be sequenced (e.g., nucleic acids); if necessary, this can be achieved by using a recording solution which is denaturing. Most preferably, UNAs are synthesized for nanopore sequencing to reduced levels of secondary structure. Using single stranded DNA, single channel recordings can be made in up to 40% formamide and at temperatures as high as 45° C. using e.g., the α-hemolysin toxin protein in a lipid bilayer. These conditions are not intended to exclude use of any other denaturing conditions. One skilled in the art of electrophysiology will readily be able to determine suitable conditions by 1) observing incorporation into the bilayer of functional channels or pores, and 2) observing transient blockades of conductance unintemipted by long-lived blockades caused by polymers becoming stuck in the channel because of secondary structure. Denaturing conditions are not always necessary for the polymerase-based methods or for double stranded DNA methods of the invention. They may not be necessary for single stranded methods either, if the pore itself is able to cause denaturation, or if the secondary structure does not interfere.

3) Concentration of Polymers in Solutions

Concentration of polymers can be rapidly and accurately assessed by using relatively low resolution recording conditions and analyzing the number of conductance blockade events in a given unit of time. This relationship should be linear and proportional (the greater the concentration of polymers, the more frequent the current blockage events), and a standardized curve can be prepared using known concentrations of polymer.

B. Principles and Techniques

1) Recording Techniques

The conductance monitoring methods of the invention rely on an established technique, single-channel recording, which detects the activity of molecules that form channels in biological membranes. When a voltage potential difference is established across a bilayer containing an open pore molecule, a steady current of ions flows through the pore from one side of the bilayer to the other. The nucleotide bases of a DNA molecule, for example, passing through or over the opening of a channel protein, disrupt the flow of ions through the pore in a predictable way. Fluctuations in the pore's conductance caused by this interference can be detected and recorded by conventional single-channel recording techniques. Under appropriate conditions, with modified nucleotides if necessary, the conductance of a pore can change to unique states in response to the specific bases in DNA.

This flux of ions can be detected, and the magnitude of the current describes the conductance state of the pore. Multiple conductance states of a channel can be measured in a single recording as is well known in the art. By recording the fluctuations in conductance of the maltoporin (LamB) pore, for example, when DNA is passed through it by phage lambda injection or over its opening by the action of a polymerase fused to the surface of the LamB protein, we estimate that a sequencing rate of 100-1000 bases/sec/pore can be achieved.

The monitoring of single ion channel conductance is an inexpensive, viable method that has been successful for the last two decades and is in very wide spread current use. It directly connects movements of single ions or channel proteins to digital computers via amplifiers and analog to digital (A to D, A/D) converters. Single channel events taking place in the range of a few microseconds can be detected and recorded (Hamill et al., 1981, Pfluegers Arch. Eur. J. Physiol., 391: 85-100). This level of time resolution ranges from just sufficient to orders of magnitude greater than the level we need, since the time frame for movement of nucleotide bases relative to the pore for the sequencing method is in the range of microseconds to milliseconds. The level of time resolution required depends on the voltage gradient or the enzyme turnover number if the nucleic acid is moved by an enzyme. Other factors controlling the level of time resolution include medium viscosity, temperature, etc.

The characteristics and conductance properties of any pore molecule that can be purified can be studied in detail using art-known methods (Sigworth et al., J. Biophys., 52:1055-1064, 1987; Heinemann et al., 1988, Biophys. J., 54: 757-64; Wonderlin et al., 1990, Biophys. J., 58: 289-97). These optimized methods are ideal for our nucleic acid sequencing application. For example, in the pipette bilayer technique, an artificial bilayer containing at least one pore protein is attached to the tip of a patch-clamp pipette by applying the pipette to a preformed bilayer reconstituted with the purified pore protein in advance. Due to the very narrow aperture diameter of the patch pipette tip (2 microns), the background noise for this technique is significantly reduced, and the limit for detectable current interruptions is about 10 microseconds (Sigworth et al., supra; Heinemann et al., 1990, Biophys. J., 57:499-514). Purified channel protein can be inserted in a known orientation into preformed lipid bilayers by standard vesicle fusion techniques (Schindler, 1980, FEBS Letters, 122:77-79), or any other means known in the art, and high resolution recordings are made. The membrane surface away from the pipette is easily accessible while recording. This is important for the subsequent recordings that involve added DNA. The pore can be introduced into the solution within the patch pipette rather than into the bath solution.

An optimized planar lipid bilayer method has recently been introduced for high resolution recordings in purified systems (Wonderlin et al., supra). In this method, bilayers are formed over very small diameter apertures (10-50 microns) in plastic. This technique has the advantage of allowing access to both sides of the bilayer, and involves a slightly larger bilayer target for reconstitution with the pore protein. This optimized bilayer technique is an alternative to the pipette bilayer technique.

Instrumentation is needed which can apply a variable range of voltages from about +400 Mv to -400 mV across the channel/membrane, assuming that the trans compartment is established to be 0 mV; a very low-noise amplifier and current injector, analog to digital (A/D) converter, data acquisition software, and electronic storage medium (e.g., computer disk, magnetic tape). Equipment meeting these criteria is readily available, such as from Axon Instruments, Foster City, Calif. (e.g., Axopatch 200 A system; pClamp 6.0.2 software).

Preferred methods of large scale DNA sequencing involve translating from base pairs to electronic signals as directly and as quickly as possible in a way that is compatible with high levels of parallelism, miniaturization and manufacture. The method should allow long stretches (even stretches over 40 kbp) to be read so that errors associated with assembly and repetitive sequence can be minimized. The method should also allow automatic loading of (possibly nonredundant) fresh sequences.

2) Channels and Pores Useful in the invention

Any channel protein which has the characteristics useful in the invention (e.g., pore sized up to about 9 nm) may be employed. Pore sizes across which polymers can be drawn may be quite small and do not necessarily differ for different polymers. Pore sizes through which a nucleic acid is drawn will be e.g., approximately 0.5-2.0 nm for single stranded DNA; 1.0-3.0 nm for double stranded DNA. These values are not absolute, however, and other pore sizes might be equally functional for the nucleic acid types mentioned above.

Non-limiting examples of bacterial pore-forming proteins which can be used in the invention include Gramicidin (e.g., Gramicidin A from *Bacillus brevis*; available from Fluka, Ronkonkoma, N.Y.); LamB (maltoporin), OmpF, OmpC, or PhoE from *Escherichia coli,* Shigella, and other Enterobacteriaceae, alpha-hemolysin (from *S. aureus*), Tsx, the F-pilus, lambda exonuclease, and mitochondrial porin (VDAC).

A modified voltage-gated channel can also be used in the invention, as long as it does not inactivate quickly, e.g., in less than about 500 msec (whether naturally or following modification to remove inactivation) and has physical parameters suitable for e.g., polymerase attachment (recombinant fusion proteins) or has a pore diameter suitable for nucleic acid passage. Methods to alter inactivation characteristics of voltage gated channels are well known in the art (see e.g., Patton, et al., Proc. Natl. Acad. Sci. USA, 89: 10905-09 (1992); West, et al., Proc. Natl. Acad. Sci. USA, 89: 10910-14 (1992); Auld, et al., Proc. Natl. Acad. Sci. USA, 87: 323-27 (1990); Lopez, et al., Neuron, 7: 327-36 (1991); Hoshi, et al., Neuron, 7: 547-56 (1991); Hoshi, et al., Science, 250: 533-38 (1990)).

Appropriately sized physical or chemical pores may be induced in a water-impermeable barrier (solid or membranous) up to a diameter of about 9 nm, which should be large enough to accommodate most polymers (either through the pore or across its opening). Any methods and materials known in the art may be used to form pores, including track etching and the use of porous membrane templates which can be used to produce pores of the desired material (e.g., scanning-tunneling microscope or atomic force microscope related methods).

Chemical channels or pores can be formed in a lipid bilayer using chemicals (or peptides) such as Nystatin, as is well known in the art of whole-cell patch clamping ("perforated patch" technique); and peptide channels such as Alamethicin.

Template-dependent nucleic acid polymerases and free nucleotides can be used as a motor to draw the nucleic acids through the channel. For example, the DNA to be sequenced is placed in one chamber; RNA polymerases, nucleotides, and optionally primers are placed in the other chamber. As the 3' end of the DNA passes through the channel (via a voltage pulse or diffusion, for example), the RNA polymerase captures and begins polymerization. If the polymerase is affixed to the chamber or is physically blocked from completely passing through the channel, the polymerase can act as a ratchet to draw the DNA through the channel.

Similarly, lambda exonuclease, which is itself shaped as a pore with a dimension similar to α-hemolysin, can operate as a motor, controlling the movement of the nucleic acid nucleic acid through the channel. The exonuclease has the added benefit of allowing access to one strand of a double stranded polymer. As the double stranded nucleic acid passes through the pore, the exonuclease grabs onto the 5' single-stranded overhang of a first strand (via endonuclease digestion or breathing of the double stranded DNA ends) and sequentially cleaves the complementary second strand at its 3' end. During the sequential cleavage, the exonuclease progresses 5' to 3' down the first strand, pulling the double stranded DNA through the channel at a controlled rate. Thus, the exonuclease can operate as a pore as well as a motor for drawing the nucleic acid nucleic acid through the channel.

To produce pores linked with polymerase or exonuclease, synthetic/recombinant DNA coding for a fusion protein can be transcribed and translated, then inserted into an artificial membrane in vitro. For example, the C-terminus of *E. coli* DNA polymerase I (and by homology, T7 DNA polymerase) is very close to the surface of the major groove of groove of the newly synthesized DNA. If the C-terminus of a polymerase is fused to the N-terminus of a pore forming protein such as colicin E1 and the colicin is inserted into an artificial membrane, one opening of the colicin pore should face the DNA's major groove and one should face the opposite side of the lipid bilayer. For example, the colicin molecule can be modified to achieve a pH optimum compatible with the polymerase as in Shiver et al. (J. Biol. Chem., 262: 14273-14281, 1987). Both pore and polymerase domains can be modified to contain cysteine replacements at points such that disulfide bridges form to stabilize a geometry that forces the pore opening closer to the major groove surface and steadies the nucleic acid as it passes the pore opening. The loops of the pore domain at this surface can be systematically modified to maximize sensitivity to changes in the DNA sequence.

C. General Considerations for Conductance Based Measurements

1) Electrical/Channel Optimization

The conductance of a pore at any given time is determined by its resistance to ions passing through the pore (pore resistance) and by the resistance to ions entering or leaving the pore (access resistance). For a pore's conductance to be altered in discrete steps, changes in one or both of these resistance factors will occur by unit values. The base pairs of a DNA molecule represent discrete units that are distinct from each other along the phosphate backbone. As long as the orientation of DNA to the pore remains relatively constant, and the membrane potential does not change, as each base pair passes by (or through) the pore, it is likely to interfere with a reproducible number of ions. Modifications made to the individual bases would influence the magnitude of this effect.

To resolve stretches of repeating identical bases accurately, and to minimize reading errors in general, it may be useful for the pore to register a distinct (probably higher) level of conductance in between the bases. This can take place naturally in the pore-polymerase system with helix rotation during polymerization, or in the phage system between entry of base pairs into the pore, or when the regions in between base pairs pass by a rate limiting site for ion flux inside the pore. Modified bases used to distinguish nucleotide identities may also contribute significantly to this issue, because they should magnify the conductance effect of the bases relative to the effect of regions in between the bases. With single strand passage through a pore, charged phosphates may punctuate the passage of each base by brief, higher conductance states. Also, if the rate of movement is constant, then punctuation between bases may not be required to resolve stretches of repeating identical bases.

Altered conductance states have been described for many channels, including some LamB mutants (Dargent et al., J. Mol. Biol., 201:497-506, 1988). A mutant may be a valuable alternative to a wild type channel protein if its fluctuation to a given state is sensitive to nucleotide bases in DNA. Alternative systems can also be developed from other channel proteins that are known to have multiple single channel conductance states. Examples of these are the alamethicin channel, which under certain conditions fluctuates through at least 20 discrete states (Taylor et al., 1991, Biophys. J., 59: 873-79), and the OmpF porin, which shows gating of its individual monomers giving rise to four discrete states (Lakey et al., 1989, Eur. J. Biochem., 186: 303-308).

Since channel events can be resolved in the microsecond range with the high resolution recording techniques available, the limiting issue for sensitivity with the techniques of our invention is the amplitude of the current change between bases. Resolution limits for detectable current are in the 0.2 pA range (1 pA=6.24 x $10^6$ ions/sec). Each base affecting pore current by at least this magnitude is detected as a separate base. It is the function of modified bases to affect current amplitude for specific bases if the bases by themselves are poorly distinguishable.

One skilled in the art will recognize that there are many possible configurations of the sequencing method described herein. For instance, lipid composition of the bilayer may include any combination of non-polar (and polar) components which is compatible with pore or channel protein incorporation. Any configuration of recording apparatus may be used (e.g., bilayer across aperture, micropipette patches, intra-vesicular recording) so long as its limit of signal detection is below about 0.5 pA, or in a range appropriate to detect monomeric signals of the nucleic acid being evaluated. If polymeric size determination is all that is desired, the resolution of the recording apparatus may be much lower.

A Nernst potential difference, following the equation

$$E_{ion} = (RT/zF)\log_e ([ion]_o / [ion]_i)$$

where $E_{ion}$ is the solvent ion (e.g., potassium ion) equilibrium potential across the membrane, R is the gas constant, T is the absolute temperature, z is the valency of the ion, F is Faraday's constant, $[ion]_o$ is the outside and $[ion]_i$ is the inside ionic concentration (or trans and cis sides of the bilayer, respectively), can be established across the bilayer to force polymers across the pore without supplying an external potential difference across the membrane. The membrane potential can be varied ionically to produce more or less of a differential or "push." The recording and amplifying apparatus is capable of reversing the gradient electrically to clear blockages of pores caused by secondary structure or cross-alignment of charged polymers.

2) Optimization of Methods

In an operating system of the invention, one can demonstrate that the number of transient blockades observed is quantitatively related to the number of nucleic acid molecules that move through the channel from the cis to the trans compartment. By sampling the trans compartment solution after observing one to several hundred transient blockades and using quantitative, competitive PCR assays (e.g., as in Piatak et al., 1993, BioTechniques, 14: 70-79) it is possible to measure the number of molecules that have traversed the channel. Procedures similar to those used in competitive PCR can be used to include an internal control that will distinguish between DNA that has moved through the channel and contaminating or aerosol DNA.

Further steps to optimize the method may include:

1. Slowing the passage of polynucleotides so that individual nucleotides can be sensed. Since the blockade durations we observed are in the millisecond range, each nucleotide in a one or two thousand monomer-long polynucleotide occupies the channel for just a few microseconds. To measure effects of individual nucleotides on the conductance, substantially reducing the velocity may offer substantial improvement. Approaches to accomplish this include: (a) increasing the viscosity of the medium, (b) establishing the lower limit of applied potential that will move polynucleotides into the channel (c) use of high processivity polymerase in the trans compartment to "pull" DNA through the pore in place of voltage gradients. Using enzymes to pull the DNA through the pore may also solve another potential problem (see 3, below).

2. Making a channel in which an individual nucleotide modulates current amplitude. While α-toxin may give rise to distinguishable current amplitudes when different mono-polynucleotides pass through the channel, 4-5 nucleotides in the strand necessarily occupy the length of its approximately 50 .ANG. long channel at any given time. Ionic current flow may therefore reflect the sum of the nucleotide effects, making it difficult to distinguish monomers. To determine current modulation attributable to individual monomers, one may use channels containing a limiting aperture that is much shorter than the full length of the overall channel. For example, one can modify α-hemolysin by standard molecular biological techniques such that portions of the pore leading to and away from the constriction are widened.

3. Enhancing movement of DNA in one direction. If a DNA molecule is being pulled through a channel by a voltage gradient, the probability of its moving backward against the gradient will be given by $e^{-(\text{energy to move against the voltage gradient}/kT)}$

where kT is energy associated with thermal fluctuations. For example, using reasonable assumptions for the effective charge density of the DNA polyelectrolyte in buffer (Manning, 1969, J. Chem. Phys., 51: 924-33), at room temperature the probability of thermal energy moving the DNA molecule backward 10Å against a 100 mV voltage gradient $e^{-4}$, or about one in fifty. Should this problem exist, some kind of ratchet mechanism, possibly a polymerase or other DNA binding protein, may be useful in the trans chamber to prevent backward movements of the DNA.

3) Advantages of Single Channel Sequencing

The length of continuous DNA sequence obtainable from the methods described herein will only be limited in certain embodiments (e.g., by the packaging limit of phage lambda heads (~50 kb) or by the size of the template containing polymerase promoter sequences). Other embodiments (e.g., voltage gradients) have no such limitation and should even make it possible to sequence DNA directly from tissue samples, since the technique is not limited to cloned DNA. Having large contiguous sequence as primary input data will substantially reduce the complexity of sequence assembly, particularly in the case of repetitive DNA. There are other applications if consistent conductance behaviors can be correlated with particular properties of given molecules (i.e. shape).

D. Specific Methods and Examples of Current Based Characterization

The following specific non-limiting examples of current based polymer characterization are presented to illustrate the method of nanopore sequencing.

1) The LamB pore

Maltoporin (LamB) is an outer membrane protein from *E. coli* that functions as a passive diffusion pore (porin) for small molecules and as a specific transport pore for passage of maltose and maltodextrins (Szmelcman et al., 1975, J. Bacteriol., 124: 112-18). It is also the receptor for bacteriophage lambda (Randall-Hazelbauer and Schwartz, 1973, J. Bacteriol. 116: 1436-1446). Three identical copies of the LamB gene product assemble to form the native pore. Each subunit (MW ~48,000) is composed of predominantly beta-structure and is a pore in itself, though it is thought that the three pores fuse into one at the periplasmic side of the membrane (Lepault et al., 1988, EMBO, J., 7: 261-68).

A protein folding model for LamB is available that predicts which portions of the mature protein reside on the external and periplasmic surfaces of the membrane (Charhit et al., 1991, J. Bacteriol., 173: 262-75). Permissive sites in the protein have been mapped to several extramembranous loops that tolerate the insertion of foreign polypeptides without significantly disrupting pore properties (Boulain et al., 1986, Mol. Gen. Genet., 205: 339-48; Charbit et al., 1986, EMBO J., 5: 3029-37; Charbit et al., 1991, supra). The LamB protein has been crystallized and a high resolution structure derived (3.1Å) (Schirmer et al., 1995, Science, 267: 512-514).

The pore properties of wild type LamB and a few mutant proteins have been studied at low resolution in planar lipid bilayer single channel recordings (Benz et al., 1986, J. Bacteriol., 165: 978-86; Benz et al., 1987, J. Membrane Biol., 100: 21-29; Dargent et al., 1987, FEBS Letters, 220: 136-42; Dargent et al., 1988, J. Mol. Biol., 201: 497-506). The pore has a very stable conductance of 150 pS in 1M NaCl, and shows selectivity for maltose and maltodextrins. These molecules effectively block conductance of the pore. One LamB mutant (Tyr$^{163} \rightarrow$ Asp) exhibits distinct sublevels of conductance (30 pS each).

The LamB pore is extremely stable, and high time resolution recordings can be made for use in this invention. The time resolution of channel conductance measurements with the conventional planar lipid bilayer technique is limited because of the background noise associated with the high electrical capacitance of bilayers formed on large diameter apertures (100-200 microns), but smaller apertures or insulated glass microelectrodes can improve the resolution of LamB channel recordings. Preferably, improved LamB conductance recordings will use the pipette bilayer technique (Sigworth et al., supra).

In another embodiment of the invention, the individual nucleotide sequence of single-stranded DNA or RNA or the individual base-pair sequence of double-stranded DNA or RNA molecules is determined using electron tunneling currents by sensing the electronic properties of the individual nucleotide bases (or base pairs) as they move past the aperture. Tunneling is a purely quantum mechanical effect that allows particles of nature to penetrate into region of space that would normally be inaccessible by the principles of Newtonian classical mechanics. When tunneling, the quantum mechanical spatial wavefunction of a particle acquires an exponential form with a decay constant that depends on the square root of the particle mass and potential barrier inhibiting the motion. For charged particles, tunneling can be observed experimentally through electrical currents associated with their transport through classically forbidden regions. The small mass of an electron enhances the penetration into these regions and, hence, electronic rather than ionic conduction is the phenomena of interest.

While electron-tunneling spectroscopy has achieved atomic scale resolution of images, these techniques have not yet produced information regarding DNA sequence. Electron tunneling methods have been limited by problems of aligning the electrode tip with a DNA molecule immobilized onto a viewing surface.

In the method of the invention, the multimeric single or double-stranded DNA or RNA molecule traverses a spatially narrow region or pore, which specifically favors the examination of the linear molecule. Tunneling is considered a particularly preferred method of monitoring the passage of DNA through the aperture because tunneling currents associated with the operation of the tunneling microscope are in the 1-10 nanoamp range, which is two or three orders of magnitude greater than ionic conduction currents.

According to this aspect of the present invention, metal electrodes are deposited on a synthetic solid-state membrane on either side of the aperture and are in electrical communication with the aperture. A protective insulating layer may be deposited on the electrodes. The surface area of the electrode in contact with the aperture is quite small, making it a sensitive probe of the changes in the DNA composition as it traverses the aperture. Membranes having an aperture of the appropriate diameter (e.g., between 2 and 4 nm) and deposited electrodes can be fabricated by methods described in the art (e.g. WO 00/78668, incorporated herein by reference).

For these types of tunneling current measurements, the aperture-containing membrane is configured in a circuit that applies a voltage bias between the tunneling electrodes and that enables measurement of the tunneling current indicative of molecular traversal between the electrodes. Connection to the membrane electrodes is made in any suitable conventional manner, e.g. by wire bonding, direct ionic contact with a fluid, or other suitable techniques.

The present invention includes in its scope systems and kits for practicing methods of nanopore sequencing and UNA generation as taught herein. Furthermore, it is recognized that variations to the methods described herein may be performed by those skilled in the art which are encompassed by the scope of the present invention as disclosed and/or claimed herein. In addition, it is recognized that experimental error/variability may occur when practicing the present invention which may deviate from the description herein.

**Claims**

1.  A method of sequencing a nucleic acid molecule comprising steps of:

    providing two separate, adjacent solutions of a medium and an interface between the two pools, the interface having a channel so dimensioned as to allow sequential nucleotide-by-nucleotide passage from one pool to the other pool of only one nucleic acid molecule at a time;
    providing a nucleic acid molecule with at least one tandem repeat of a nucleotide sequence to be determined, wherein the nucleic acid molecule is synthesized using a circular template;
    placing the nucleic acid molecule in one of the two pools; and

taking measurements as each of the nucleotides of the nucleic acid molecule passes through the channel so as to determine the sequence of the nucleic acid molecule.

2. A method as claimed in claim 1 wherein the nucleic acid has a reduced level of secondary structure.

3. A method as claimed in claim 1 or 2, wherein the nucleic acid molecule is enzymatically synthesized, and wherein the nucleic acid molecule contains modified nucleotides that reduce secondary structure in the nucleic acid molecule.

4. A method as claimed in claim 1, 2 or 3, wherein the nucleic acid is single-stranded DNA.

5. A method as claimed in claim 1, 2 or 3, wherein the nucleic acid is single-stranded RNA.

6. A method as claimed in claim 1, 2 or 3, wherein the nucleic acid molecule is double-stranded and wherein the nucleic acid molecule is enzymatically synthesized.

7. A method as claimed in claim 6, wherein the double-stranded nucleic acid is DNA.

8. A method as claimed in claim 6, wherein the double-stranded nucleic acid is RNA.

9. A method as claimed in any of claims 3 to 8, wherein the nucleic acid molecule contains modified adenosine and modified thymine which are not able to form a base pair, wherein the modified adenosine is capable of forming a base pair with unmodified thymine, and wherein the modified thymine is capable of forming a. base pair with unmodified adenosine.

10. A method as claimed in any of claims 3 to 9, wherein the nucleic acid molecule contains modified guanosine and modified cytosine which are not able to form a base pair, wherein the modified guanosine is capable of forming a base pair with unmodified cytosine, and wherein the modified cytosine is capable of forming a base pair with unmodified guanosine.

11. A method as claimed in any of claims 3 to 10, wherein the nucleic acid molecule contains 2-aminoadenosine, 2-thiothymidine, inosine and pyrrolopyrimidine.

12. A method as claimed in any of claims 3 to 10, wherein the nucleic acid molecule contains 2-aminoadenosine and 2-thiothymidine.

13. A method as claimed in any preceding claim, wherein the circular template is single-stranded.

14. A method as claimed in of any of claims 1 to 12, wherein the circular template is double-stranded.

15. A method as claimed in of any preceding claim, wherein the nucleic acid polymer interacts with an inner surface of the channel.

16. A method as claimed in any preceding claim, wherein the medium is electrically conductive.

17. A method as claimed in any preceding claim, further comprising applying a voltage across the interface.

18. A method as claimed in claim 17, wherein ionic flow between the two pools is measured.

19. A method as claimed in claim 18, wherein the duration of ionic flow blockage is measured.

20. A method as claimed in claim 18, wherein the amplitude of ionic flow blockage is measured.

21. A method as claimed in any preceding claim, further comprising providing a polymerase or exonuclease in one of the two pools, wherein the polymerase or exonuclease draws the nucleic acid polymer through the channel.

22. A method as claimed in any preceding claim, wherein the medium is an aqueous solution.

23. A method as claimed in any preceding claim, further comprising analysing the nucleic acid molecules by electron tunnelling.

**Patentansprüche**

1.  Ein Verfahren zum Sequenzieren eines Nukleinsäuremoleküls, das folgende Schritte umfasst:

    Bereitstellen zweier getrennter, benachbarter Lösungen eines Mediums und einer Grenzfläche zwischen den zwei Pools, wobei die Grenzfläche einen Kanal aufweist, der dahin gehend bemessen ist, einen sequentiellen, Nukleotid um Nukleotid erfolgenden Durchtritt immer nur eines Nukleinsäuremoleküls zu einem Zeitpunkt von einem Pool zu dem anderen Pool zu ermöglichen;
    Bereitstellen eines Nukleinsäuremoleküls mit zumindest einer Tandemwiederholung einer zu bestimmenden Nukleotidsequenz, wobei das Nukleinsäuremolekül unter Verwendung einer kreisrunden Matrize synthetisiert wird;
    Platzieren des Nukleinsäuremoleküls in einen der beiden Pools; und
    Vornehmen von Messungen, während jedes der Nukleotide des Nukleinsäuremoleküls durch den Kanal tritt, um die Sequenz des Nukleinsäuremoleküls zu bestimmen.

2.  Ein Verfahren gemäß Anspruch 1, bei dem die Nukleinsäure einen verringerten Sekundärstruktur-Pegel aufweist.

3.  Ein Verfahren gemäß Anspruch 1 oder 2, bei dem das Nukleinsäuremolekül enzymatisch synthetisiert wird und bei dem das Nukleinsäuremolekül modifizierte Nukleotide enthält, die die Sekundärstruktur in dem Nukleinsäuremolekül reduzieren.

4.  Ein Verfahren gemäß Anspruch 1, 2 oder 3, bei dem die Nukleinsäure einsträngige DNA ist.

5.  Ein Verfahren gemäß Anspruch 1, 2 oder 3, bei dem die Nukleinsäure einsträngige RNA ist.

6.  Ein Verfahren gemäß Anspruch 1, 2 oder 3, bei dem das Nukleinsäuremolekül doppelsträngig ist und bei dem das Nukleinsäuremolekül enzymatisch synthetisiert wird.

7.  Ein Verfahren gemäß Anspruch 6, bei dem die doppelsträngige Nukleinsäure DNA ist.

8.  Ein Verfahren gemäß Anspruch 6, bei dem die doppelsträngige Nukleinsäure RNA ist.

9.  Ein Verfahren gemäß einem der Ansprüche 3 bis 8, bei dem das Nukleinsäuremolekül modifiziertes Adenosin und modifiziertes Thymin enthält, die nicht in der Lage sind, ein Basenpaar zu bilden, bei dem das modifizierte Adenosin in der Lage ist, mit unmodifiziertem Thymin ein Basenpaar zu bilden, und bei dem das modifizierte Thymin in der Lage ist, mit unmodifiziertem Adenosin ein Basenpaar zu bilden.

10. Ein Verfahren gemäß einem der Ansprüche 3 bis 9, bei dem das Nukleinsäuremolekül modifiziertes Guanosin und modifiziertes Cytosin enthält, die nicht in der Lage sind, ein Basenpaar zu bilden, bei dem das modifizierte Guanosin in der Lage ist, mit unmodifiziertem Cytosin ein Basenpaar zu bilden, und bei dem das modifizierte Cytosin in der Lage ist, mit unmodifiziertem Guanosin ein Basenpaar zu bilden.

11. Ein Verfahren gemäß einem der Ansprüche 3 bis 10, bei dem das Nukleinsäuremolekül 2-Aminoadenosin, 2-Thiothymidin, Inosin und Pyrrolopyrimidin enthält.

12. Ein Verfahren gemäß einem der Ansprüche 3 bis 10, bei dem das Nukleinsäuremolekül 2-Aminoadenosin und 2-Thiothymidin enthält.

13. Ein Verfahren gemäß einem der vorhergehenden Ansprüche, bei dem die kreisförmige Matrize einsträngig ist.

14. Ein verfahren gemäß einem der Ansprüche 1 bis 12, bei dem die kreisförmige Matrize doppelsträngig ist.

15. Ein Verfahren gemäß einem der vorhergehenden Ansprüche, bei dem das Nukleinsäurepolymer mit einer Innenoberfläche des Kanals interagiert.

**16.** Ein Verfahren gemäß einem der vorhergehenden Ansprüche, bei dem das Medium elektrisch leitfähig ist.

**17.** Ein Verfahren gemäß einem der vorhergehenden Ansprüche, das ferner ein Anlegen einer Spannung über die Grenzfläche umfasst.

**18.** Ein Verfahren gemäß Anspruch 17, bei dem ein Ionenfluss zwischen den zwei Pools gemessen wird.

**19.** Ein Verfahren gemäß Anspruch 18, bei dem die Dauer der Ionenflussblockierung gemessen wird.

**20.** Ein Verfahren gemäß Anspruch 18, bei dem die Amplitude der Ionenflussblockierung gemessen wird.

**21.** Ein Verfahren gemäß einem der vorhergehenden Ansprüche, das ferner ein Bereitstellen einer Polymerase oder Exonuklease in einem der beiden Pools umfasst, wobei die Polymerase oder Exonuklease das Nukleinsäurepolymer durch den Kanal zieht.

**22.** Ein Verfahren gemäß einem der vorhergehenden Ansprüche, bei dem das Medium eine wässrige Lösung ist.

**23.** Ein Verfahren gemäß einem der vorhergehenden Ansprüche, das ferner ein Analysieren der Nukleinsäuremoleküle mittels Elektronentunnelung umfasst.

**Revendications**

**1.** Procédé de séquençage d'une molécule d'acide nucléique comprenant les étapes consistant à :

■ fournir deux solutions adjacentes distinctes d'un milieu et une interface entre les deux groupes, l'interface ayant un canal dimensionné de manière à permettre le passage séquentiel nucléotide par nucléotide d'un groupe à l'autre de seulement une molécule d'acide nucléique à la fois ;
■ fournir une molécule d'acide nucléique avec au moins une répétition en tandem d'une séquence nucléotidique à déterminer, où la molécule d'acide nucléique est synthétisée en utilisant une matrice circulaire ;
■ placer la molécule d'acide nucléique dans l'un des deux groupes ; et
■ prendre les mesures lorsque chacun des nucléotides de la molécule d'acide nucléique passe à travers le canal de manière à déterminer la séquence de la molécule d'acide nucléique.

**2.** Procédé selon la revendication 1, dans lequel l'acide nucléique a un niveau réduit de structure secondaire.

**3.** Procédé selon la revendication 1 ou 2, dans lequel la molécule d'acide nucléique est synthétisée de manière enzymatique, et dans lequel la molécule d'acide nucléique contient des nucléotides modifiés qui réduisent la structure secondaire dans la molécule d'acide nucléique.

**4.** Procédé selon la revendication 1, 2 ou 3, dans lequel l'acide nucléique est de l'ADN simple brin.

**5.** Procédé selon la revendication 1, 2 ou 3, dans lequel l'acide nucléique est de l'ARN simple brin.

**6.** Procédé selon la revendication 1, 2 ou 3, dans lequel la molécule d'acide nucléique est double brin et dans lequel la molécule d'acide nucléique est synthétisée de manière enzymatique.

**7.** Procédé selon la revendication 6, dans lequel l'acide nucléique double brin est de l'ADN.

**8.** Procédé selon la revendication 6, dans lequel l'acide nucléique double brin est de l'ARN.

**9.** Procédé selon l'une quelconque des revendications 3 à 8, dans lequel la molécule d'acide nucléique contient de l'adénosine modifiée et de la thymine modifiée qui ne sont pas capables de former une paire de bases, dans lequel l'adénosine modifiée est capable de former une paire de bases avec de la thymine non modifiée, et dans lequel la thymine modifiée est capable de former une paire de bases avec de l'adénosine non modifiée.

**10.** Procédé selon l'une quelconque des revendications 3 à 9, dans lequel la molécule d'acide nucléique contient de la guanosine modifiée et de la cytosine modifiée qui ne sont pas capables de former une paire de bases, dans lequel la guanosine modifiée est capable de former une paire de bases avec de la cytosine non modifiée, et dans lequel la cytosine modifiée est capable de former une paire de bases avec de la guanosine non modifiée.

11. Procédé selon l'une quelconque des revendications 3 à 10, dans lequel la molécule d'acide nucléique contient de la 2-aminoadénosine, de la 2-thiothymidine, de l'inosine et de la pyrrolo-pyrimidine.

12. Procédé selon l'une quelconque des revendications 3 à 10, dans lequel la molécule d'acide nucléique contient de la 2-aminoadénosine et de la 2-thiothymidine.

13. Procédé selon l'une quelconque des revendications précédentes, dans lequel la matrice circulaire est simple brin.

14. Procédé selon l'une quelconque des revendications 1 à 12, dans lequel la matrice circulaire est double brin.

15. Procédé selon l'une quelconque des revendications précédentes, dans lequel le polymère d'acide nucléique interagit avec une surface interne du canal.

16. Procédé selon l'une quelconque des revendications précédentes, dans lequel le milieu est électriquement conductif.

17. Procédé selon l'une quelconque des revendications précédentes, comprenant en outre l'application d'une tension à travers l'interface.

18. Procédé selon la revendication 17, dans lequel le flux ionique entre les deux groupes est mesuré.

19. Procédé selon la revendication 18, dans lequel la durée du blocage du flux ionique est mesurée.

20. Procédé selon la revendication 18, dans lequel l'amplitude du blocage du flux ionique est mesurée.

21. Procédé selon l'une quelconque des revendications précédentes, comprenant en outre la fourniture d'une polymérase ou d'une exonucléase dans l'un des deux groupes, dans lequel la polymérase ou l'exonucléase attire le polymère d'acide nucléique à travers le canal.

22. Procédé selon l'une quelconque des revendications précédentes, dans lequel le milieu est une solution aqueuse.

23. Procédé selon l'une quelconque des revendications précédentes, comprenant en outre l'analyse des molécules d'acide nucléique par effet tunnel électronique.

# Figure 1

### Serial Reading Through a Single Pore

### Parallel Reading Through Multiple Pores

# Figure 2

PCR

Circularize using
DNA Ligase

Generate Single
Stranded DNA

Single-Stranded
or
Double-Stranded
DNA Template

Circularize using
Splint & DNA Ligase

Add Primer

Rolling Circle
Primer Extension

Figure 3

# Figure 4

PCR

Phage RNA Polymerase
Promoter

Generate Single
Stranded DNA

Circularize using
Splint & DNA Ligase

Single-
Stranded
DNA Template

Add Promoter
Oligo

Rolling Circle
Primer Extension
with Phage RNA
Polymerase

# Figure 5

PCR

Phage RNA Polymerase
Promoter

Circularize using
DNA Ligase

Rolling Circle
Primer Extension
with Phage RNA
Polymerase

EP 1 225 234 B1

# Figure 6

RCA DNA

RCA UNA

Figure 7

Primer Extension
with UNA NTPs (o)

Disruption of
Intramolecular Structures

EP 1 225 234 B1

Figure 8A

Figure 8B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6015714 A, Baldarelli
- US 5795782 A, Church
- US 5714320 A
- WO 9217484 A, E. T. Kool

- US 5854033 A
- WO 9918241 A
- US 358141 A
- WO 0078668 A

**Non-patent literature cited in the description**

- **KASIANOWICZ et al.** *Proc. Natl. Acad. Sci. USA.,* 1996, vol. 93, 13770-3
- **TESSIER et al.** *Biochem,* 1986, vol. 158, 171-178
- **G. PRAKASH et al.** *J. Am. Chem. Soc.,* 1992, vol. 114, 3523-3527
- **E. KANAYA et al.** *Biochemistry,* 1986, vol. 25, 7423-7430
- **L. BEAUCAGE et al.** *Tetrahedron Lett.,* 1981, vol. 22, 1859
- **S. A. SCARINGE et al.** *Nucleic Acids Res.,* 1990, vol. 18, 5433
- **JOHNSON.** *Annual Review in Biochemistry,* 1993, vol. 62, 685-713
- **GOODMAN et al.** *Critical Review in Biochemistry and Molecular Biology,* 1993, vol. 28, 83-126
- **CHAMBERLIN ; RYAN.** The Enzymes. Academic Press, 1982, 87-108
- **SWITZER et al.** *Biochemistry,* 1993, vol. 32, 10489-10496
- **LUTZ et al.** *Nucleic Acids Research,* 1996, vol. 24, 1308-1313
- **LITTLE et al.** *J. Biol Chem.,* 1967, vol. 242, 672
- **HIGUCHI ; OCHAMN.** *Nucleic Acids Research,* 1989, vol. 17, 5865
- **CHAMBERLIN ; RYAN.** The Enzymes. Qacademic Press, 1982, 87-108
- **MELTON et al.** *Nucleic Acids Research,* 1984, vol. 12, 7035
- **MILLIGAN ; UHLENBECK.** *Methods in Enzymology,* 1989, vol. 180A, 51-62
- **MIZUSAWA et al.** *Nucleic Acids Research,* 1986, vol. 14, 1319
- **BAILLY ; WARING.** *Nucleic Acids Research,* 1996, vol. 23, 885
- **RACKWITZ ; SCHEIT.** *Eur. J. Biochem.,* 1977, vol. 72, 191
- **BLOOM et al.** *Biochemistry,* 1993, vol. 32, 11247-11258

- **LAW et al.** *Biochemistry,* 1996, vol. 35, 12329-12337
- **SAENGER.** Principles of Nucleic Acid Structure. Springer-Verlag, 1983
- **WAGNER et al.** *Science,* 1993, vol. 260, 1510
- **KUTYAVIN et al.** *Biochemistry,* 1996, vol. 35, 11170
- **WOO et al.** *Nucleic Acids Research,* 1996, vol. 24, 2470
- **ISRAELACHVILI.** Intermolecular and Surface Forces. Academic Press, 1992
- **ASHKIN et al.** *Oppt. Lett.,* 1986, vol. 11, 288
- **KUO ; SHEETZ.** *Science,* 1993, vol. 260, 232
- **SVOBODA et al.** *Nature,* 1993, vol. 365, 721
- **QUATE, F.** *Surf. Sci.,* 1994, vol. 299, 980
- **MATE et al.** *Phys. Rev. Lett.,* 1987, vol. 59, 1942
- **FRISBIE et al.** *Science,* 1994, vol. 265, 71
- **WARREN.** *Ann. Rev. Microbiol.,* 1980, vol. 34, 137-58
- **HAMILL et al.** *Pfluegers Arch. Eur. J. Physiol.,* 1981, vol. 391, 85-100
- **SIGWORTH et al.** *J. Biophys.,* 1987, vol. 52, 1055-1064
- **HEINEMANN et al.** *Biophys. J.,* 1988, vol. 54, 757-64
- **WONDERLIN et al.** *Biophys. J.,* 1990, vol. 58, 289-97
- **HEINEMANN et al.** *Biophys. J.,* 1990, vol. 57, 499-514
- **SCHINDLER.** *FEBS Letters,* 1980, vol. 122, 77-79
- **PATTON et al.** *Proc. Natl. Acad. Sci. USA,* 1992, vol. 89, 10905-09
- **WEST et al.** *Proc. Natl. Acad. Sci. USA,* 1992, vol. 89, 10910-14
- **AULD et al.** *Proc. Natl. Acad. Sci. USA,* 1990, vol. 87, 323-27
- **LOPEZ et al.** *Neuron,* 1991, vol. 7, 327-36
- **HOSHI et al.** *Neuron,* 1991, vol. 7, 547-56
- **HOSHI et al.** *Science,* 1990, vol. 250, 533-38

- **SHIVER et al.** *J. Biol. Chem.,* 1987, vol. 262, 14273-14281
- **DARGENT et al.** *J. Mol. Biol,* 1988, vol. 201, 497-506
- **TAYLOR et al.** *Biophys. J.,* 1991, vol. 59, 873-79
- **LAKEY et al.** *Eur. J. Biochem.,* 1989, vol. 186, 303-308
- **PIATAK et al.** *BioTechniques,* 1993, vol. 14, 70-79
- **MANNING.** *J. Chem. Phys.,* 1969, vol. 51, 924-33
- **SZMELCMAN et al.** *J. Bacteriol.,* 1975, vol. 124, 112-18
- **RANDALL-HAZELBAUER ; SCHWARTZ.** *J. Bacteriol.,* 1973, vol. 116, 1436-1446
- **LEPAULT et al.** *EMBO, J.,* 1988, vol. 7, 261-68
- **CHARHIT et al.** *J. Bacteriol.,* 1991, vol. 173, 262-75
- **BOULAIN et al.** *Mol. Gen. Genet.,* 1986, vol. 205, 339-48
- **CHARBIT et al.** *EMBO J,* 1986, vol. 5, 3029-37
- **SCHIRMER et al.** *Science,* 1995, vol. 267, 512-514
- **BENZ et al.** *J. Bacteriol.,* 1986, vol. 165, 978-86
- **BENZ et al.** *J. Membrane Biol.,* 1987, vol. 100, 21-29
- **DARGENT et al.** *FEBS Letters,* 1987, vol. 220, 136-42